(19) Europäisches Patentamt
European Patent Office
Office européen des brevets

(11) **EP 2 715 310 B1**

(12) **FASCICULE DE BREVET EUROPEEN**

(45) Date de publication et mention
de la délivrance du brevet:
**27.04.2022  Bulletin 2022/17**

(21) Numéro de dépôt: **12731087.8**

(22) Date de dépôt: **29.05.2012**

(51) Classification Internationale des Brevets (IPC):
**G01N 1/28** *(2006.01)*        **G01N 33/68** *(2006.01)*

(52) Classification Coopérative des Brevets (CPC):
**G01N 1/38; G01N 33/4833; G01N 33/6851;**
G01N 2001/2866; G01N 2001/2893

(86) Numéro de dépôt international:
**PCT/FR2012/051205**

(87) Numéro de publication internationale:
**WO 2012/164221 (06.12.2012 Gazette 2012/49)**

(54) **PROCÉDÉ DE DÉTECTION ET DE QUANTIFICATION D'UNE MOLÉCULE CIBLE DANS UN TISSU**

VERFAHREN ZUM NACHWEIS UND ZUR QUANTIFIZIERUNG EINES ZIELMOLEKÜLS IN EINEM GEWEBE

METHOD FOR DETECTING AND QUANTIFYING A TARGET MOLECULE IN A TISSUE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **31.05.2011  FR 1154731**

(43) Date de publication de la demande:
**09.04.2014  Bulletin 2014/15**

(73) Titulaire: **Imabiotech**
**59655 Villeneuve D'ascq (FR)**

(72) Inventeurs:
 • **STAUBER, Jonathan**
  **59800 Lille (FR)**
 • **BONNEL, David**
  **62840 Laventie (FR)**

(74) Mandataire: **Cabinet Becker et Associés**
**25, rue Louis le Grand**
**75002 Paris (FR)**

(56) Documents cités:
 • **J. SA. BECKER ET AL: "Elemental imaging mass spectrometry of thin sections of tissues and analysis of brain proteins in gels by laser ablation inductively coupled plasma mass spectrometry", PHYSICA STATUS SOLIDI (C), vol. 4, no. 6, 1 mai 2007 (2007-05-01), pages 1775-1784, XP55033030, ISSN: 1610-1634, DOI: 10.1002/pssc.200675226**
 • **DOMINIC HARE ET AL: "Quantitative elemental bio-imaging of Mn, Fe, Cu and Zn in 6-hydroxydopamine induced Parkinsonism mouse models", METALLOMICS, vol. 1, no. 1, 1 janvier 2009 (2009-01-01) , page 53, XP055013049, ISSN: 1756-5901, DOI: 10.1039/b816188g**
 • **BASAK KÜKRER KALETAS ET AL: "Sample preparation issues for tissue imaging by imaging MS", PROTEOMICS, vol. 9, no. 10, 1 mai 2009 (2009-05-01), pages 2622-2633, XP055011676, ISSN: 1615-9853, DOI: 10.1002/pmic.200800364**
 • **DREXLER D M ET AL: "Utility of imaging mass spectrometry (IMS) by matrix-assisted laser desorption ionization (MALDI) on an ion trap mass spectrometer in the analysis of drugs and metabolites in biological tissues", JOURNAL OF PHARMACOLOGICAL AND TOXICOLOGICAL METHODS, ELSEVIER, NEW YORK, NY, US, vol. 55, no. 3, 1 mai 2007 (2007-05-01), pages 279-288, XP025321039, ISSN: 1056-8719, DOI: 10.1016/J.VASCN.2006.11.004 [extrait le 2007-03-23]**

- NOTARI S ET AL: "Determination of anti-HIV drug concentration in human plasma by MALDI-TOF/TOF", JOURNAL OF CHROMATOGRAPHY B: BIOMEDICAL SCIENCES & APPLICATIONS, ELSEVIER, AMSTERDAM, NL, vol. 833, no. 1, 20 mars 2006 (2006-03-20) , pages 109-116, XP025122412, ISSN: 1570-0232, DOI: 10.1016/J.JCHROMB.2006.02.010 [extrait le 2006-03-20]
- DOMINIC J. HARE ET AL: "Three-dimensional elemental bio-imaging of Fe, Zn, Cu, Mn and P in a 6-hydroxydopamine lesioned mouse brain", METALLOMICS, vol. 2, no. 11, 1 janvier 2010 (2010-01-01), page 745, XP055011675, ISSN: 1756-5901, DOI: 10.1039/c0mt00039f
- ZABET-MOGHADDAM M ET AL: "Qualitative and quantitative analysis of low molecular weight compounds by ultraviolet matrix-assisted laser desorption/ionization mass spectrometry using ionic liquid matrices", RAPID COMMUNICATIONS IN MASS SPECTROMETRY, HEYDEN, LONDON, GB, vol. 18, no. 2, 30 janvier 2004 (2004-01-30), pages 141-148, XP002592602, ISSN: 0951-4198, DOI: 10.1002/RCM.1293 [extrait le 2003-12-18]

**Description**

Domaine de l'invention

L'invention concerne l'utilisation d'une gamme de dilution d'une molécule pour un tissu pour la quantification de ladite molécule dans ledit tissu, par exemple par spectrométrie de masse. La gamme de dilution selon l'invention tient compte du comportement spécifique de la molécule à quantifier dans le tissu d'intérêt. Plus particulièrement, l'invention fournit un procédé permettant la détection et/ou la quantification d'une molécule cible directement à la surface d'un tissu, en utilisant l'imagerie par spectrométrie de masse, et notamment l'imagerie MALDI. L'invention concerne également un procédé de validation d'un procédé de détection et/ou de quantification, ainsi qu'un procédé pour contrôler la qualité et/ou la reproductibilité d'un procédé de détection et/ou de quantification.

D'une manière générale, l'invention trouve des applications dans tout domaine où la détection et/ou la quantification d'une molécule dans un tissu est utile/nécessaire. L'invention trouve par exemple des applications dans le domaine pharmaceutique pour étudier la distribution et la pharmacocinétique d'un médicament dans différents tissus biologiques. De même, l'invention trouve des applications dans le domaine biomédical, par exemple pour détecter, identifier et tracer un biomarqueur donné, dans un tissu pathologique à un instant donné. L'invention trouve également des applications dans le domaine de l'agrochimie, par exemple pour évaluer la toxicité et la dégradation d'une molécule telle qu'un produit phytosanitaire dans des plantes.

Etat de la technique

Différentes techniques sont actuellement utilisées pour détecter et identifier une molécule dans un échantillon, notamment un tissu. Par exemple, la spectrométrie de masse est une technique largement connue et utilisée en analyse chimique et biochimique, pour détecter et identifier des molécules d'intérêt dans un échantillon. Depuis quelques années, l'imagerie moléculaire par spectrométrie de masse, telle que l'imagerie MALDI, s'est développée, permettant de visualiser la distribution de molécules d'intérêt directement sur des coupes de tissus biologiques. L'imagerie MALDI, de par sa très grande sensibilité, permet de visualiser simultanément la distribution d'un très grand nombre de molécules différentes directement à la surface des tissus. Dans le domaine pharmaceutique, cette technologie permet par exemple de comparer la distribution d'une molécule dans différents organes à différents temps de traitement.

Cependant, dans le cas où une quantification de la molécule ainsi détectée à l'instant t est souhaitée, il est nécessaire de coupler cette technique d'imagerie à une analyse chimique quantitative, par une méthode classique ou instrumentale. Cette seconde étape de quantification est source d'erreurs de manipulation et d'interprétation. De plus, elle ne permet pas une corrélation directe entre la présence de la molécule d'intérêt et sa distribution quantitative dans le tissu.

Par ailleurs, une molécule donnée à une concentration donnée n'émet pas un signal de même intensité selon le tissu dans lequel elle est détectée. De même, deux molécules différentes à une concentration identique dans un tissu donné présentent une intensité de signal différente.

Ce phénomène peut s'expliquer par l'existence d'un coefficient d'extinction tissulaire (TEC), ou effet tissu, ou encore effet surpression d'ion, propre à chaque molécule et chaque tissu. Le TEC est représentatif de la perte ou du gain d'intensité du signal d'une molécule donnée suivant le tissu et/ou sa localisation dans le tissu, par rapport au signal de ladite molécule sur un support d'analyse, inerte. Le TEC est dépendant de plusieurs facteurs, et notamment de la nature du tissu (animal ou végétal), de l'environnement chimique, du traitement chimique ou non du tissu etc. L'existence de ce TEC rend difficile l'interprétation de l'intensité du signal émis par une molécule dans un tissu lors d'une analyse, notamment en spectrométrie de masse.

Les articles suivants sont considérés état de technique: Becker et al. "Elemental imaging mass spectrometry of thin sections of tissues and analysis of brain proteins in gels by laser ablation inductively coupled plasma mass spectrometry", PHYSICA STATUS SOLIDI (C), 4(6), 2007;

Hare et al. "Quantitative elemental bio-imaging of Mn, Fe, Cu and Zn in 6-hydroxydopamine induced Parkinsonism mouse models", METALLOMICS, 1(1), 2009.

Exposé de l'invention

Un procédé permettant de préparer une gamme de dilution, ou gamme étalon, d'une molécule donnée pour un tissu donné est divulgué. La gamme étalon est préparée en utilisant une coupe d'une reconstitution du tissu d'intérêt à partir d'un tissu fragmenté. Les inventeurs ont découvert qu'une molécule donnée se comporte de façon équivalente dans un tel tissu reconstitué et dans le tissu intègre correspondant. Dans le cas d'une analyse par spectrométrie de masse, le spectre de masse de la molécule dans le tissu reconstitué est sensiblement identique au spectre de masse de la même molécule, à la même concentration, dans le tissu intègre correspondant. De même, dans le cas d'une analyse par fluorescence, l'intensité du signal fluorescent émis par la molécule marquée dans le tissu reconstitué est sensiblement identique à l'intensité du signal fluorescent émis par la même molécule marquée, à la même concentration, dans le tissu intègre correspondant. Aussi, en utilisant une gamme étalon selon l'invention pour une analyse quantitative d'une molécule donnée dans un tissu donné, il n'est pas nécessaire de tenir compte du TEC. Le signal obtenu lors de l'analyse de ladite molécule dans ledit tissu peut directement être corrélé aux signaux de la gamme étalon correspondante.

L'invention propose également un procédé pour détecter et/ou quantifier une molécule cible dans un tissu, quel que soit le mode de détection et notamment par ionisation, radioactivité ou fluorescence. Par exemple, le procédé selon l'invention utilise la spectrométrie de masse, ou l'imagerie par spectrométrie de masse, permettant une acquisition automatisée d'un signal lié au spectre de masse de la molécule, directement sur une coupe du tissu, afin de reconstruire des images de la distribution et de la quantité de ladite molécule cible dans le tissu. Pour cela, selon l'invention, on utilise une gamme de dilution de la molécule cible, ou d'une molécule présentant des caractéristiques physico-chimiques similaires, réalisée en utilisant une coupe d'un tissu reconstitué à partir d'un tissu identique au tissu à analyser. Une quantification précise de la molécule dans le tissu est alors possible par comparaison directe avec les résultats de la gamme de dilution.

L'invention propose également un procédé de validation d'un procédé de détection et/ou de quantification d'une molécule cible dans un tissu en utilisant une coupe de tissu. Le procédé de validation selon l'invention permet de vérifier pour un tissu donné et une molécule donnée, que les résultats d'analyse obtenus pour ladite molécule directement sur une coupe dudit tissu seront représentatifs dudit tissu, indépendamment de l'effet tissu.

L'invention propose également un procédé pour contrôler la qualité et/ou la reproductibilité d'un procédé de détection et/ou de quantification d'une molécule cible dans un tissu en utilisant une coupe de tissu. Un tel procédé permet de suivre les variabilités expérimentales et/ou d'en tenir compte notamment en normalisant les valeurs obtenues pour chaque analyse par rapport à ce contrôle qualité afin par exemple de faire de la quantification relative de(s) composé(s) d'intérêt entre différentes analyses.

L'invention divulgue un procédé de préparation d'une gamme de dilution d'au moins une molécule étalon pour un tissu, comprenant les étapes consistant à :

(i) broyer un tissu pour obtenir un broyat de tissu ;
(ii) mélanger la molécule étalon à un premier échantillon du broyat de tissu de l'étape (i), ladite molécule étalon étant à une première concentration connue ;
(iii) conditionner le premier échantillon de broyat de tissu issu de l'étape (ii) de manière à pouvoir couper ledit échantillon de broyat de tissu conditionné ;
(iv) répéter les étapes (ii) et (iii) avec au moins un second échantillon du broyat de tissu de l'étape (i) et une seconde concentration connue de la molécule étalon, différente de la première concentration ;
(v) analyser au moins une coupe de chacun des échantillons de broyat de tissu conditionné issu de l'étape (iii), de manière à obtenir un signal représentatif de la quantité de molécule étalon dans le tissu pour chacune des concentrations de ladite molécule étalon.

Le procédé peut s'appliquer à tout tissu biologique, qu'il soit d'origine animale ou végétale. Par tissu, on entend d'une manière générale un ensemble de cellules de même origine et regroupées en un ensemble fonctionnel pour concourir à une même fonction. Dans certains cas, un tissu peut s'entendre d'un organe ou d'une partie d'organe.

La molécule étalon peut être un peptide, un polypeptide, une protéine, un acide aminé, un acide nucléique, un lipide, un métabolite, une petite molécule telle qu'une drogue etc. Plus généralement, la molécule étalon peut être toute molécule active sur le plan pharmaceutique, biologique ou autre. Selon l'analyse devant être réalisée à l'étape (v), la molécule étalon peut être non marquée ou marquée, et notamment radiomarquée ou marquée avec une molécule fluorescente, telle que la GFP (« Green Fluorescente Protéine »).

Lors de l'étape (i) de broyage, le tissu peut être fragmenté de manière à former un broyat plus ou moins fin, notamment liquide, semi-liquide, pâteux etc. Le broyat de tissu peut ne pas être homogène et par exemple comporter des fragments de tissu de différentes tailles. Il est possible, au besoin, d'ajouter une solution d'homogénéisation au broyat obtenu, et notamment une solution aqueuse, de manière à diluer ledit broyat et le rendre moins compact. La solution d'homogénéisation peut être ajouter en amont, en aval ou pendant l'étape de broyage.

Le broyage peut être effectué par tout moyen, notamment manuel ou mécanique. Par exemple, le broyat de tissu est obtenu par cisaillement à l'aide d'un mixeur, ou par écrasement à l'aide d'un pilon.

Le tissu utilisé à l'étape (i) est avantageusement un organe, tel qu'un rein, un foie etc. On utilise alors indifféremment un organe entier ou une portion seulement dudit organe pour réaliser la gamme étalon. Le tissu peut être broyé avant d'être subdivisé en au moins autant d'échantillons de broyat de tissu qu'il y a de points de dilution dans la gamme étalon à réaliser. Il est autrement possible de procéder à la division dudit tissu avant le broyage, chaque échantillon étant individuellement broyé. Il est également possible d'utiliser un ou plusieurs organes différents, entiers ou non, pour chacun des points de dilution de la gamme étalon, les organes utilisés pour la réalisation d'une même gamme étant à chaque fois de nature et d'origine identiques. Par exemple, pour la réalisation d'une gamme étalon d'une molécule dans un foie de rat, comprenant quatre points de dilution, ou concentrations, on utilise pour chaque concentration un nouveau foie de rat.

Des préparations de la molécule étalon à au moins deux concentrations différentes sont réalisées. Par exemple, la molécule étalon est mise en suspension dans une solution de solubilisation (aqueuse ou contenant un solvant), à différentes concentrations connues. Une même quantité de solution est alors ajoutée à chacun des échantillons de broyat de tissu, chacune ayant une concentration différente et connue en ladite molécule étalon. Dans un autre exemple, la molécule étalon est mise en suspension dans une solution de solubilisation, des quantités différentes de ladite solution étant ajoutées à chacun des échantillons de broyat de tissu, de manière à ce que chaque échantillon de broyat de tissu issu de l'étape (ii) présente une concentration différente et connue en ladite molécule étalon. Préférentiellement, la gamme de dilution comporte entre 2 et 10 points de dilution.

Avantageusement, on prévoit avant l'étape (iii) de conditionnement une étape d'homogénéisation, afin d'obtenir une répartition homogène de la molécule étalon dans l'échantillon de broyat de tissu. Cette étape d'homogénéisation peut par exemple se faire à l'aide d'un mélangeur tel qu'un Vortex.

Dans un exemple de réalisation, les étapes (i), (ii) et le cas échéant l'étape d'homogénéisation se font successivement ; il est bien entendu possible de conduire tout ou partie de ces étapes simultanément. Par exemple, on broie le tissu avec la solution contenant la molécule étalon.

L'étape (iii) de conditionnement consiste en un traitement et/ou une mise en forme de l'échantillon de broyat de tissu, pour permettre sa découpe afin d'obtenir au moins une coupe dudit broyât.

Par exemple, le conditionnement comporte une étape de congélation de l'échantillon de broyat de tissu. Pour cela, l'échantillon de broyat de tissu est par exemple disposé dans un moule, dont la forme peut être quelconque, l'ensemble étant ensuite congelé par toute technique appropriée. Par exemple, ledit moule est plongé dans l'azote liquide, afin de provoquer une congélation instantanée de l'ensemble de la masse, puis maintenu à -18°C jusqu'à l'étape de découpe.

Dans un autre exemple, l'étape de conditionnement consiste en un enrobage dudit échantillon de broyat de tissu, notamment dans de la paraffine, de la résine ou de la gélatine. Par enrobage, on entend un revêtement qui entoure entièrement l'échantillon de broyat de tissu. L'étape d'enrobage peut notamment se faire en utilisant un moule.

Bien entendu, l'étape de congélation peut être suivie d'une étape d'enrobage.

Le choix du conditionnement peut notamment dépendre de l'état de l'échantillon de broyat de tissu. Par exemple, si le broyat de tissu issu de l'étape (ii) est liquide, on choisira avantageusement de congeler l'échantillon de broyat de tissu pour permettre la découpe ultérieure. Dans le cas où le broyat consiste en un hachis grossier du tissu, ou en une pâte compacte, un enrobage dans de la paraffine ou autre peut suffire à permettre sa découpe.

Dans un mode de réalisation, il est possible d'utiliser un même moule pour conditionner tout ou partie des échantillons de broyat de tissu d'une même gamme étalon. Dans ce cas, le moule comporte différents casiers, pour permettre une séparation physique entre les différents échantillons. Avantageusement, les cloisons délimitant les casiers sont amovibles et/ou dans un matériau susceptible d'être découpé aux mêmes conditions que les échantillons de broyat de tissu. Ainsi, il est possible de réaliser des coupes à partir du contenu du moule, qui comportent chacune la succession d'échantillons de broyat de tissu de la gamme de dilution en cours de réalisation. Préférentiellement, les échantillons de broyat de tissu sont disposés par concentrations croissantes (ou décroissantes) successives dans les casiers successifs.

Une fois conditionnés, les échantillons de broyat de tissu peuvent être indifféremment maintenus dans les conditions du conditionnement, par exemple congelés, ou être conservés dans des conditions garantissant le maintien de leur intégrité (par exemple à une température de +5°C environ), dans leurs moules ou démoulés, jusqu'à leur découpe et/ou leur utilisation dans l'étape (v) d'analyse.

Avantageusement, l'échantillon de broyat de tissu issu de l'étape (iii) présente une masse volumique identique à celle d'un tissu correspondant intègre. De même, avantageusement, l'échantillon de broyat de tissu issu de l'étape (iii) présente un coefficient d'extinction tissulaire (TEC) identique à celui d'un tissu correspondant intègre.

Par masse volumique, on entend la densité du tissu ou d'échantillon de broyat de tissu.

Par tissu intègre, on entend un tissu identique au tissu utilisé pour réaliser les échantillons de broyat de tissu, qui conserve les qualités/propriétés inhérentes dudit tissu.

L'étape (v) d'analyse est conduite sur des coupes des échantillons de broyat de tissu, préalablement réalisées à partir des échantillons de broyat conditionnés. Toute technologie permettant une analyse moléculaire sur une coupe de tissu peut être utilisée pour l'étape (v), comme la spectrométrie de masse, l'analyse par fluorescence, l'autoradiographie etc.

D'une manière générale, une coupe s'entend d'une section d'un tissu (reconstitué ou intègre). Aussi, toutes les dimensions de coupes compatibles avec la l'analyse moléculaire et notamment quantitative, par exemple dans un spectromètre ou un microscope, peuvent être utilisées. Avantageusement, les coupes présentent une épaisseur comprise entre $2\mu m$ et $50\mu m$, et notamment environ $20\mu m$. L'épaisseur s'entend de la dimension s'étendant perpendiculairement au plan de la coupe.

Selon des exemples de mise en œuvre du procédé, les coupes peuvent être traitées avant l'étape d'analyse. Par exemple, il est possible de procéder à une dessiccation de la coupe obtenue à partir d'un échantillon de broyat de tissu préalablement congelé ou enrobé. Dans le cas d'une coupe issue d'un échantillon de broyat de tissu préalablement congelé, il est possible de procéder à un cryo-desséchage de ladite coupe. Par exemple, la coupe est placée à -18°C pendant une nuit avant l'étape d'analyse.

Les coupes de tissu peuvent être réalisées à partir d'échantillons de broyat de tissu encore congelés, ou sur lesdits échantillons une fois décongelés. Avantageusement, pour la réalisation d'une gamme de dilution donnée, les mêmes conditionnements et traitement(s) ultérieur(s) éventuel(s) sont appliqués à tous les échantillons de broyat de tissu. Préférentiellement, toutes les coupes utilisées pour la réalisation d'une gamme étalon donnée sont d'épaisseur sensiblement identique.

Avantageusement, les coupes d'échantillons de broyat de tissu sont déposées sur un support, tel qu'une lame, permettant leur introduction au sein d'un spectromètre, d'un microscope, ou autre. Préférentiellement, toutes les coupes utilisées pour la réalisation d'une gamme étalon donnée sont disposées sur des supports identiques pour la conduite de l'analyse.

D'une manière générale, les mêmes conditions d'analyse sont avantageusement appliquées à tous les échantillons de broyat de tissu d'une même gamme de dilution. Par conditions d'analyse, on entend notamment les réglages et paramétrages de l'appareil utilisé pour l'analyse, et les conditions de préparation des échantillons, comme les étapes de lavage éventuelles, le dépôt d'une matrice dans le cas d'une analyse par spectrophotométrie, etc.

Une fois le signal représentatif de la quantité de la molécule obtenu pour chacune des coupes d'échantillon de broyat de tissu de la gamme, il est possible de réaliser une courbe de calibration représentative de la gamme de dilution.

Par exemple, dans le cas d'une analyse par spectrométrie de masse, on choisit un même signal associé au spectre de masse de la molécule cible pour tous les points de dilution, pour tracer la courbe de calibration. Le signal peut notamment être l'intensité du pic, l'aire du pic ou le ratio signal/bruit dudit spectre de masse.

Dans le cas d'une analyse par autoradiographie, on utilise l'intensité de la radioactivité émise par la molécule étalon radiomarquée, par exemple au carbone 14. Dans le cas d'une analyse par fluorescence, on utilise l'intensité de l'émission lumineuse de la molécule étalon marquée par une molécule fluorescente.

Dans un mode de réalisation du procédé, on réalise au moins deux gammes étalons différentes en même temps.

Par exemple, au moins deux molécules étalons différentes sont ajoutées à l'étape (ii) dans chacun des échantillons du broyat de tissu, l'analyse de l'étape (v) étant conduite pour chacune des molécules étalons de manière à obtenir le signal représentatif de la quantité en la molécule étalon de chacune des molécules étalons dans le tissu, pour chacune des concentrations.

Chaque molécule étalon est ajoutée dans un échantillon de broyat de tissu donné à une concentration donnée, qui peut être différente de la concentration en l'autre molécule dans ledit échantillon de broyat de tissu.

La gamme de dilution peut être utilisée pour calculer la plus petite quantité de molécule étalon détectable dans le tissu avec un niveau de confiance donné, ou LOD (« Limit of détection »). Pour cela, on identifie sur la gamme de dilution le plus petit signal détectable dans la limite de détection, c'est-à-dire présentant une valeur signal/bruit supérieure ou égale à 3.

De même, la gamme étalon peut être utilisée pour calculer la plus petite quantité de molécule étalon quantifiable dans le tissu avec un niveau de confiance donné, ou LOQ (« Limit of quantification »). Cette valeur (signal/bruit) est fixée au niveau international comme devant être égale à trois fois la valeur le LOD (LOQ=3LOD) et être au moins égale à 10.

La gamme de dilution peut également être utilisée pour évaluer l'influence de l'épaisseur des coupes et/ou de la nature de la matrice et/ou du mode de dépôt de ladite matrice sur la détection et/ou quantification de la molécule. Il est ainsi possible de déterminée l'épaisseur de coupe optimum et/ou la matrice la plus appropriée en fonction du tissu et/ou de la molécule.

Les gammes de dilution obtenues selon le procédé peuvent également être utilisées pour quantifier une molécule cible dans un tissu lors d'une analyse dudit tissu.

L'invention a donc pour objet une utilisation d'une gamme de dilution d'une molécule étalon dans un tissu reconstitué pour quantifier une molécule cible dans un tissu d'intérêt identique au tissu reconstitué de la gamme de dilution.

De même, l'invention a pour objet un procédé de détection et de quantification d'au moins une molécule cible dans au moins un tissu d'intérêt comprenant les étapes suivantes :

a) analyser une coupe du tissu d'intérêt, de manière à obtenir un signal représentatif de la quantité de molécule cible dans ladite coupe ;
b) déterminer la quantité de molécule cible dans ledit tissu en utilisant une gamme de dilution d'une molécule étalon dans un tissu reconstitué identique au tissu d'intérêt.

La gamme de dilution utilisée est obtenue selon les étapes (i) à (v) décrites ci-dessus en utilisant un tissu identique au tissu d'intérêt pour réaliser le broyat de tissu.

Le tissu s'entend d'au moins une partie d'au moins un tissu biologique (d'origine végétale ou animale).

Dans un mode de réalisation, il est possible de quantifier au moins une molécule cible dans un échantillon tissulaire comportant plusieurs tissus, et notamment plusieurs tissus adjacents dans un organisme. Par exemple, la coupe de l'échantillon tissulaire peut consister en une coupe d'un animal entier ou d'une portion d'un animal, comportant au moins un organe ou au moins une portion d'organe. Notamment l'analyse sur une coupe d'un animal entier peut permettre de comparer, sur un même échantillon, la distribution d'une ou plusieurs molécules cibles dans différents tissus dudit animal. Dans ce cas, pour déterminer la quantité d'une molécule cible donnée dans chacun des tissus de l'échantillon tissulaire, on utilise à chaque fois une gamme de dilution de ladite molécule cible spécifique du tissu considéré.

L'étape a) d'analyse de la molécule cible peut se faire par toute méthode d'analyse, comme la spectrométrie de masse, et notamment en utilisant la spectrométrie de masse directe (MS) ou en tandem ($MS^n$, MRM, SRM), l'analyse par fluorescence, l'autoradiographie etc.

Le procédé selon l'invention peut être avantageusement utilisé avec la spectrométrie de masse. Dans ce cas, il est possible d'utiliser différentes sources d'ionisation, MALDI (Désorption-Ionisation Laser Assistée par Matrice), LDI (Désorption-Ionisation Laser), LESA (Liquid extraction Surface Analysis), LAESI (Laser Ablation Electrospray Ionization), DESI (Désorption-Ionisation par Electrospray), NanoDESI, etc, combinée à différents types d'analyseurs, TOF (Temps de Vol), Orbitrap, FTICR (Résonance Cyclotronique des Ions à Transformée de Fourier), etc. Cette technique d'imagerie permet de quantifier la molécule cible directement sur la carte de densité ionique obtenue pour l'échantillon tissulaire, correspondant à la répartition spatiale de la molécule cible dans ledit échantillon tissulaire. On peut en effet reporter le signal obtenu sur ladite carte de densité ionique sur la gamme de dilution correspondante.

Certaines techniques de spectrométrie de masse, telles que MALDI ou ME-SIMS, nécessitent de recouvrir préalablement une coupe de l'échantillon tissulaire à analyser d'une matrice comportant de petites molécules organiques absorbant dans l'UV. Cette matrice permet la désorption et l'ionisation des molécules présentes sur l'échantillon.

Le procédé selon l'invention peut être utilisé quelle que soit la matrice choisie. Ces matrices se présentent sous forme solide (cristallisation sur l'échantillon) ou liquide et sont dites ioniques ou non. Le choix de la matrice s'effectue selon la gamme de masse analysée. Elles sont le plus souvent préparées extemporanément dans un mélange solvant-solution aqueuse.

Plusieurs modes de dépôt de la matrice sont possibles, et notamment le dépôt manuel à l'aide d'une pipette, qui permet de déposer un volume précis de matrice directement sur le tissu. Il également possible de déposer la matrice par spray ou nébulisation, la matrice étant sprayée ou nébulisée directement sur l'échantillon tissulaire à l'aide d'un robot ou manuellement. De même, un dépôt par microgouttelettes par lequel la matrice est spottée sur l'échantillon tissulaire via des systèmes piézo-électriques, acoustique ou pousse-seringue peut être envisagé. Il est également possible de déposer la matrice par tamisage, afin de déposer la matrice sous forme solide.

Les paramètres expérimentaux, comme la gamme de masse et/ou l'intensité laser, sont avantageusement fixés de manière à optimiser la détection de la molécule cible en termes d'intensité, de sensibilité et de résolution.

On procède alors à l'acquisition du signal représentatif de la quantité de molécule cible dans ladite coupe.

Dans le cas où l'analyse consiste en l'acquisition du spectre de masse, différentes caractéristiques spectrales peuvent être utilisées comme signal à l'étape b), et notamment l'intensité des pics du spectre de masse, le rapport signal sur bruit (S/N), l'aire du pic, etc. Bien entendu, la caractéristique spectrale utilisé pour quantifier la molécule cible est la même que la caractéristique spectrale utilisé pour la préparation de la gamme étalon. Et plus généralement, la caractéristique du signal prise en compte pour quantifier la molécule cible est la même que la caractéristique du signal utilisée pour la préparation de la gamme étalon.

Dans certaines conditions, il est par ailleurs possible de normaliser la ou les caractéristiques spectrales à partir d'un facteur de normalisation déterminée par la technologie.

Par exemple, dans le cas où le procédé selon l'invention utilise l'imagerie par spectrométrie de masse de type MALDI, on prévoit une étape d'évaluation de l'homogénéité du dépôt de matrice sur l'échantillon. En effet, le signal correspondant à la matrice utilisée peut renseigner quant à la qualité/uniformité du dépôt de ladite matrice. Les défauts de matrice à la surface de l'échantillon peuvent ensuite être corrélés à la non-détection ou à la perte d'intensité du signal de la molécule cible dans l'échantillon considéré.

L'évaluation de l'homogénéité de la matrice peut se faire selon des critères qualitatifs, en observant au microscope optique l'homogénéité du dépôt à la surface de l'échantillon, et/ou selon des critères quantitatifs, en suivant les variations du signal relatif à la matrice elle-même sur l'échantillon.

En ce qui concerne les critères qualitatifs, il faut s'assurer que la matrice a été déposée le plus uniformément possible sur la surface considérée, qu'il n'existe pas de zones vide de matrice et que sa cristallisation est optimale.

Pour l'évaluation quantitative de l'homogénéité du dépôt de matrice, on considère la matrice comme une molécule propre dont le signal est détecté au même titre que le signal de la molécule cible, au moment de l'analyse d'un échantillon. Le signal de la molécule de matrice est ensuite comparé à son signal de référence. Le signal de référence de la matrice correspond dans ce cas au signal émis par la matrice sur un dépôt de matrice de référence, c'est-à-dire sur un échantillon et sur un support d'analyse utilisés spécifiquement pour la mesure du signal de référence de la matrice.

Par ces étapes additionnelles, on valide et normalise le signal de la molécule cible pour tenir compte de la variation de la qualité du dépôt de matrice pouvant jouer sur les caractéristiques spectrales de cette dernière.

Cette prise en compte de l'effet matrice peut être particulièrement avantageuse dans le cas où on souhaite suivre l'évolution de la présence d'une molécule cible dans le temps, la qualité du dépôt de matrice pouvant varier d'un échantillon à l'autre.

La gamme étalon utilisée peut être réalisée à l'aide d'une molécule étalon identique à la molécule cible. Il est également possible d'utiliser une molécule étalon différente de la molécule cible, et présentant des propriétés physico-chimiques similaires. Il est également possible d'utiliser la molécule cible marquée d'un isotope comme molécule étalon pour la préparation de la gamme de dilution.

Selon les méthodes d'analyse utilisées, la molécule cible peut être marquée. Par exemple, dans le cas d'une analyse de fluorescence, la molécule cible est marquée par une molécule fluorescente. De même, dans le cas d'une analyse par autoradiographie, la molécule cible est radiomarquée.

Le procédé selon l'invention peut être utilisé pour l'analyse de toutes sortes de molécules, comme par exemple des peptides, des polypeptides, des protéines, des acides aminés, des acides nucléiques, des lipides, des métabolites, etc., et, en général, pour toute molécule active sur le plan pharmaceutique ou autre, et notamment, dans le cas d'une analyse par spectrométrie de masse, pour toute molécule qui peut être ionisée.

Dans le cas où la molécule cible est une protéine de poids moléculaire élevé, il est possible d'effectuer un prétraitement enzymatique de la molécule cible, afin de la cliver en plusieurs peptides. La détection et la quantification sont alors réalisées pour au moins un des peptides issus de ladite digestion enzymatique, représentatif de ladite protéine. Par exemple, on peut utiliser de la trypsine, afin de cliver la protéine cible en plusieurs peptides préalablement identifiés.

De même, il est possible de traiter le tissu à analyser avec au moins un solvant et/ou au moins un détergent avant l'étape de détection, de manière à éliminer des molécules responsables de bruit de fond. Par exemple, un lavage au chloroforme permet de retirer certaines classes de lipides. Un lavage à l'éthanol permet quant à lui une meilleure détection des molécules de masses faibles. Ces deux lavages permettent avantageusement de retirer certaines molécules, notamment lipidiques, favorisant ainsi la détection de nouveaux ions directement sur l'échantillon tissulaire.

Le procédé selon l'invention peut également être utilisé pour quantifier au moins deux molécules cibles différentes sur un même tissu et/ou sur un même échantillon tissulaire comprenant plusieurs tissus, simultanément ou non. On utilise alors des gammes de dilutions différentes spécifiques chacune de la molécule cible considérée et du tissu considéré.

De même, une même molécule cible peut être détectée et quantifiée simultanément sur différents tissus, en analysant par spectrométrie de masse, ou autre, un échantillon tissulaire comportant plusieurs tissus. Dans ce cas, on utilise pour chacun des tissus de l'échantillon, par exemple des organes dans une coupe d'animal entier, la gamme de dilution réalisée pour ladite molécule cible dans le tissu/organe correspondant.

L'invention propose également un procédé de validation d'un procédé de détection et/ou de quantification d'au moins une molécule cible dans un tissu à partir d'une coupe dudit tissu, comprenant les étapes consistant à :

(x) broyer un tissu identique au tissu auquel le procédé de détection et/ou de quantification doit être appliqué, pour obtenir un broyat de tissu ;
(xi) mélanger une molécule étalon à au moins deux échantillons du broyat de tissu de l'étape (x), ladite molécule étalon étant à une même concentration connue pour les au moins deux échantillons de broyat de tissu ;
(xii) conditionner les échantillons de broyat de tissu issus de l'étape (xi) de manière à pouvoir couper lesdits échantillons de broyat de tissu conditionné ;
(xiii) analyser au moins une coupe de chacun des échantillons de broyat de tissu conditionné de l'étape (xii), de manière à obtenir un signal représentatif de la molécule étalon pour chacune des coupes ;
(xiv) comparer entre eux les signaux obtenus pour chacune des coupes.

Un tel procédé permet de vérifier, notamment en amont d'un procédé de détection et/ou de quantification, qu'un tissu reconstitué donné, c'est-à-dire un broyat de tissu conditionné, est représentatif du tissu correspondant intègre. Ce procédé permet d'évaluer la sensibilité du procédé de détection et/ou quantification selon l'invention pour une molécule donnée et un échantillon donné, notamment au moyen de la LOD.

Avantageusement, l'étape (xi) est conduite sur au moins trois échantillons de broyat de tissu.

La comparaison des signaux réalisée à l'étape (xiv) permet avantageusement d'évaluer la reproductibilité de la détection et/ou de la quantification d'une molécule donnée dans un tissu donné, en calculant le coefficient de variation RSD (« relative standard déviation »), donné en décimal ou en pourcentage.

Bien entendu, tous les modes de réalisation particuliers du broyat de tissu, de conditionnement, de préparation de la molécule étalon, d'analyse etc., de même que la nature du tissu et/ou de la molécule étalon, tels qu'exposés ci-dessus pour la préparation de la gamme de dilution peuvent s'appliquer pour la réalisation du procédé de validation.

L'invention propose également un procédé de contrôle de la qualité et/ou de la reproductibilité d'un procédé de détection et/ou de quantification d'au moins une molécule cible dans un tissu d'intérêt à partir d'une coupe dudit tissu, comprenant les étapes consistant à :

(xx) broyer un tissu identique au tissu d'intérêt, pour obtenir un broyat de tissu ; (xxi) mélanger une concentration connue d'une molécule étalon à un échantillon du broyat de tissu de l'étape (xx) ;
(xxii) conditionner l'échantillon de broyat de tissu issu de l'étape (xxi) de manière à pouvoir couper ledit échantillon de broyat de tissu conditionné ;
(xxiii) pour chaque coupe du tissu d'intérêt à analyser, analyser simultanément une coupe de l'échantillon de broyat de tissu conditionné de l'étape (xxii), de manière à obtenir à chaque fois un signal représentatif de la molécule étalon pour la coupe de l'échantillon de broyat de tissu conditionné de l'étape (xxii) et un signal représentatif de la molécule cible pour le tissu d'intérêt ;
(xxiv) comparer entre eux les signaux obtenus pour chacune des coupes de l'échantillon de broyat de tissu conditionné de l'étape (xxii).

Ainsi, selon l'invention une coupe de tissu dopé, et notamment d'organe, de sang, plasma, sérum, urine, tissu végétal, etc., contenant un ou plusieurs composés peut être utilisée comme contrôle qualité pour chaque analyse effectuée dans le cadre d'une étude de détection et/ou de quantification, et/ ou pour le suivi des performances d'un instrument analytique.

Le tissu d'intérêt s'entend du tissu dans lequel la détection et/ou quantification doit se faire.

L'étape (xxiii) est avantageusement réalisée en déposant sur un même support, et notamment sur une même lame, la coupe du tissu d'intérêt à analyser et la coupe de l'échantillon de broyat de tissu dopé/conditionné. Les mêmes conditions de préparation du support (par exemple la matrice utilisée, la qualité de son dépôt) et d'analyse sont ainsi appliquées aux deux coupes.

La variation du signal obtenue à l'étape (xxiv) peut être normalisée, par exemple en utilisant la moyenne de pondération correspondante, pour tenir compte des variations des paramètres tels que l'effet matrice.

Un tel procédé peut par exemple servir à la quantification relative d'une molécule par simple comparaison du signal d'une coupe à une autre, au suivi de l'évolution de la biodisponibilité d'une molécule dans le temps etc.

C'est donc également un objet de l'invention de proposer un procédé de quantification relative d'au moins une molécule cible dans un tissu d'intérêt, ledit procédé comprenant les étapes consistant à :

(xxx) broyer un tissu identique au tissu d'intérêt, pour obtenir un broyat de tissu ; (xxxi) mélanger une concentration connue d'une molécule étalon à un échantillon du broyat de tissu de l'étape (xxx) ;
(xxxii) conditionner l'échantillon de broyat de tissu issu de l'étape (xxxi) de manière à pouvoir couper ledit échantillon de broyat de tissu conditionné ;
(xxxiii) pour chaque coupe du tissu d'intérêt à analyser, analyser simultanément une coupe de l'échantillon de broyat de tissu conditionné de l'étape (xxxii), de manière à obtenir à chaque fois un signal représentatif de la molécule étalon pour la coupe de l'échantillon de broyat de tissu conditionné de l'étape (xxxii) et un signal représentatif de la molécule cible pour le tissu d'intérêt ;
(xxxiv) calculer, pour chaque coupe du tissu d'intérêt à analyser, le ratio signal de la molécule cible dans le tissu d'intérêt sur signal de la molécule étalon dans le tissu reconstitué ;
(xxxv) comparer entre eux les ratios obtenus pour chaque coupe du tissu d'intérêt pour obtenir une quantification relative de la molécule cible.

Bien entendu, tous les modes de réalisation particuliers du broyat de tissu, de conditionnement, de préparation de la molécule étalon, d'analyse etc., de même que la nature du tissu et/ou de la molécule étalon, tels qu'exposés ci-dessus pour la préparation de la gamme de dilution peuvent s'appliquer pour la réalisation du procédé de contrôle de la qualité et/ou de la reproductibilité selon l'invention, du procédé de quantification relative ou absolue et du procédé d'évaluation de la plus petite quantité d'une molécule cible détectable et/ou quantifiable dans un tissu selon l'invention.

Selon l'invention, la normalisation des données sources, c'est-à-dire les corrélations entre un signal d'une molécule cible dans un tissu et la concentration correspondante à partir d'une gamme de dilution d'une molécule étalon dans un tissu reconstitué identique, en vue de la quantification, peuvent être effectuées au moyen d'un programme d'ordinateur intégrant tout ou partie de ces facteurs.

Ce programme d'ordinateur, ou logiciel de retraitement des données, peut avantageusement pondérer ces valeurs par l'effet de matrice ou de l'étalon (isotopique ou non) lors du traitement de l'image en cas d'imagerie par spectrométrie de masse.

Avantageusement, un programme d'ordinateur selon l'invention peut comparer l'intensité de la molécule cible sur le tissu d'intérêt avec l'intensité de la molécule étalon sur le tissu reconstitué, de manière à aboutir à un ratio permettant d'effectuer une quantification relative du composé d'intérêt entre différents tissus par comparaison des ratios obtenus pour chacun d'eux.

L'invention divulgue un support de données lisible par ordinateur comprenant des instructions exécutables par l'ordinateur, comme par exemple la lecture de données brutes issues de l'analyse par spectrométrie de masse, et/ou la détermination de la courbe d'étalonnage, et/ou la normalisation des données brutes par ces derniers afin d'obtenir une valeur quantitative de la molécule cible. Avantageusement, ces instructions exécutables par l'ordinateur sont adaptées pour permettre à un système informatique d'exécuter au moins l'étape b) du procédé de quantification selon l'invention et/ou l'étape (xxiv) du procédé de contrôle de la qualité/reproductibilité d'un procédé de détection/quantification, et le cas échéant l'étape de pondération utilisant les résultats selon l'étape (xxiv), de même que l'étape (xxxiv) et/ou l'étape (xxxv) du procédé de quantification relative selon l'invention.

Le support de données comporte avantageusement au moins une gamme étalon d'au moins une molécule cible dans au moins deux tissus différents. Préférentiellement, dans le cas d'échantillons biologiques, tels qu'une coupe d'un animal entier, la base de données comporte la gamme de dilution d'au moins une molécule cible dans les différents tissus dudit échantillon.

Le support de données peut également comporter une base de données du signal de référence d'au moins une matrice utilisée en imagerie par spectrométrie de masse. Ainsi, il est possible de tenir compte de l'effet matrice lors de l'analyse de l'échantillon par imagerie mettant en œuvre le support de données.

Brève description des figures

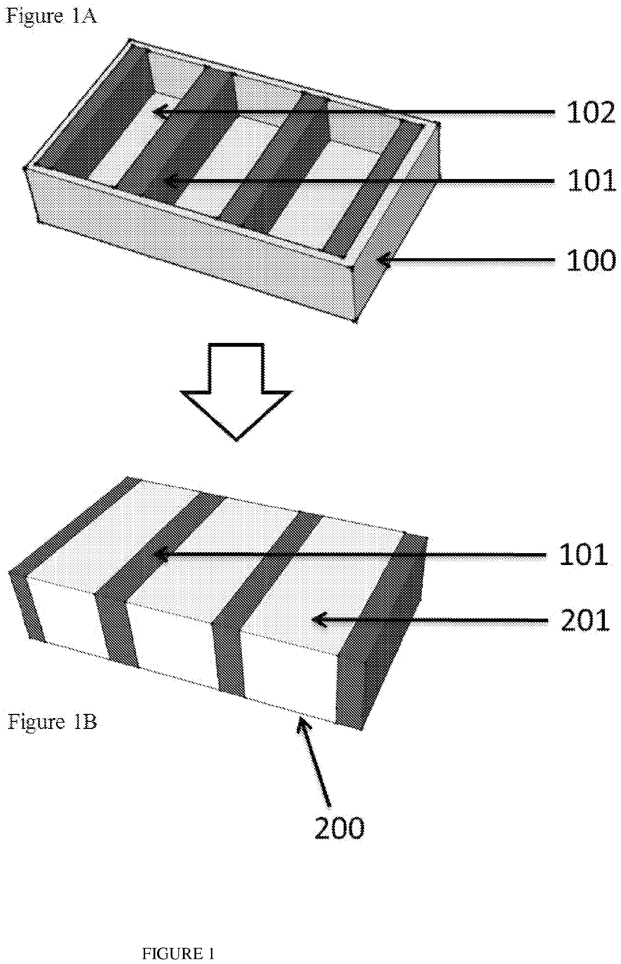

Figures 1A et 1B. Une représentation schématique d'un exemple de moule (figure 1A) à plusieurs compartiments, pouvant être utilisé lors de la mise en œuvre du procédé de préparation d'une gamme de dilution selon la description, chaque compartiment pouvant recevoir un échantillon de broyat de tissu dopé avec une concentration spécifique de molécule étalon ; et une représentation schématique des échantillons de broyat de tissu conditionnés et démoulés après congélation dans le moule (figure 1B), chaque échantillon de broyat de tissu étant séparé d'un échantillon adjacent par une cloison.

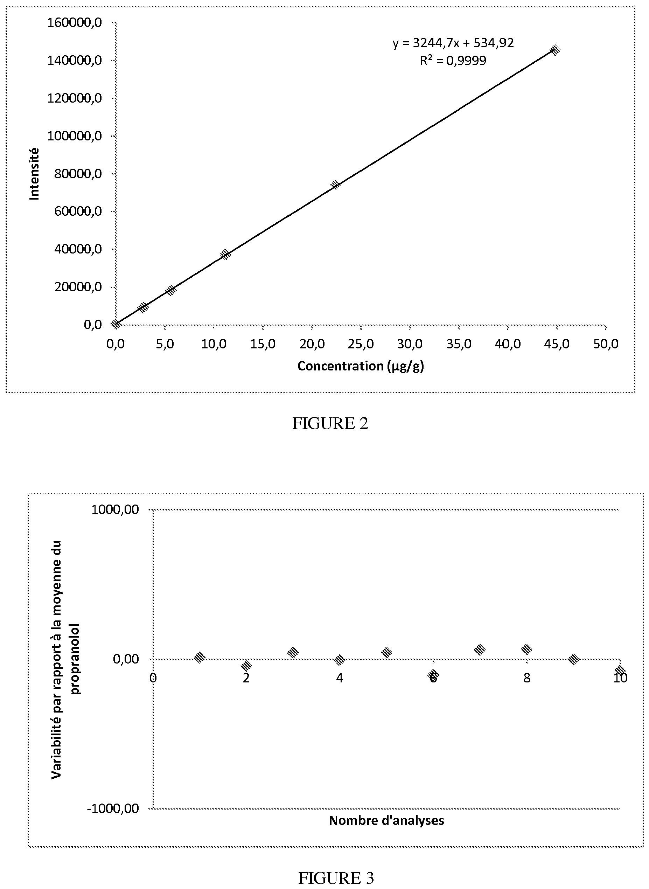

Figure 2 : Une courbe de calibration d'une molécule étalon (propranolol) dans un tissu (rein) obtenue selon le procédé de préparation de la description, représentant la concentration ($\mu$g/g - axe des abscisses) en propranolol en fonction de l'intensité émise par le propranolol (axe des ordonnées) en spectrométrie de masse.

Figure 3 : Une droite de pondération montrant la variabilité analytique en MSI mesurée à l'aide de contrôles qualité de type tissus de rein reconstitués et dopés avec une même concentration de propranolol.

EXEMPLES

**Matériel et méthode**

Matériel

Du propranolol de Sigma-Aldrich a été utilisé pour réaliser une solution de propranolol à $1.10^{-10}$ mol/$\mu$l.

Six sous-volumes précis différents de cette solution sont prélevés, qui seront chacun ajoutés à un échantillon de broyat de tissu, de manière à obtenir une concentration dans les échantillons de 0, 2.8, 5.6, 11.2, 22.4 et 44.8$\mu$g/g (tableau 1).

Echantillons de broyat de tissu

Des souris mâles de type Swiss de poids 25-40g de chez Charles River (France) ont été utilisées.

Pour la préparation de la gamme de dilution, deux reins d'une souris contrôle (c'est-à-dire n'ayant subi aucun traitement particulier) sont prélevés. Les deux reins sont broyés mécaniquement à l'aide d'un scalpel, dans un récipient préalablement stérilisé et déposé sur de la glace. Une fois le broyat de rein obtenu, on repartit de manière approximative ledit broyat dans six tubes de 1,5 ml, et chacun des six échantillons de broyat de tissu est pesé (tableau 1).

On ajoute à chacun des échantillons de broyat de tissu un des sous-volumes particulier de la solution de propranolol.

Le tableau 1 ci-dessous reprend l'ensemble des données spécifiques des six échantillons de broyat de rein.

Tableau 1 : Préparation des échantillons de broyat de rein utilisés pour la réalisation d'une gamme de dilution représentative du propranolol dans le rein.

| Numéro d'échantillon de broyat de rein | Poids homogénat (g) | Volume de propranolol ($\mu$l) d'une solution de propranolol à $1.10^{-10}$ mol/$\mu$l ajouté par échantillon | Concentration en propranolol finale dans l'échantillon ($\mu$g/g) |
|---|---|---|---|
| 1 | 0.7 | 61.7 | 2.8 |
| 2 | 0.4 | 66.4 | 5.6 |
| 3 | 0.7 | 246.8 | 11.2 |
| 4 | 0.4 | 265.8 | 22.4 |
| 5 | 0.4 | 637.8 | 44.8 |
| 6 | 0.5 | 0.0 | 0.0 |

Préparation des tissus reconstitués

Chaque tube contenant un échantillon de broyat de rein est vortexé pendant 1 heure à température ambiante, de manière à répartir de manière homogène le propranolol dans lesdits échantillons.

On utilise un moule 100 en aluminium, tel que celui représenté à la figure 1A, munis de 7 cloisons 101 transversales de manière à ménager six compartiments 102 s'étendant successivement dans une plus grande dimension dudit moule (trois compartiments seulement sont ménagés dans le moule de la figure 1A). Le moule est placé dans de l'azote liquide de manière à congeler rapidement les broyat de rein. Le moule est ensuite placé pendant 1 heure à -80°C.

Le bloc 200 de tissus reconstitués, ou conditionnés, (c'est-à-dire les échantillons de broyat de rein congelés) est ensuite démoulé (figure 1B). Les tissus reconstitués 201 sont coupés à l'aide d'un cryostat refroidi à -22°C sur une épaisseur de 20$\mu$m. Selon les besoins, on maintient sur une même coupe tous les tissus 201 de la gamme séparés les uns des autres par les cloisons 101, ou on sépare la portion correspondant au(x) tissu(s) reconstitués souhaités, du reste de la coupe.

EXEMPLE 1 : VALIDATION DU MODELE « TISSU RECONSTITUE »

Dans cet exemple, on cherche à démontrer que des tissus reconstitués, formés par des broyat de tissus qui ont été congelés, forment un modèle représentatif du tissu intègre correspondant.

Pour cela, on a mesuré et comparé la masse volumique et le coefficient d'extinction moléculaire (TEC) d'un rein reconstitué selon l'invention et d'un rein intègre.

Dix coupes du tissu reconstitué N°6 (contrôle, tableau 1) sont individualisées et placées chacune sur un support (lame ITO2).

On réalise également dix coupes de tissu de 20$\mu$m à partir d'un rein intègre prélevé sur une autre souris témoin, maintenu à température ambiante. Chaque coupe est disposée sur un support (lame ITO3).

Les supports sont scannés simultanément dans un scanneur HP Scanjet G4010 à 2400dpi et l'image est enregistrée au format jpeg.

La masse volumique moyenne des coupes de rein reconstitué et des coupes de rein intègre est alors calculée, comme expliqué dans le tableau 2 ci-dessous. Plus précisément, la valeur moyenne de la surface, de la hauteur, du volume et du poids du rein reconstitué est calculée à partir des valeurs obtenues pour chacune des dix coupes du tissu reconstitué contrôle. De même, la valeur moyenne de la surface, de la hauteur, du volume et du poids du rein intègre est calculée à partir des valeurs obtenues pour chacune des dix coupes correspondantes.

On constate que les masses volumiques des deux tissus sont sensiblement identiques, puisqu'elles ne diffèrent que de 0,04 mg/mm$^3$ (tableau 2).

Afin de calculer le TEC pour le tissu reconstitué et pour le tissu intègre, de la matrice MALDI DHB (40mg/mL méthanol/TFA 0.1% 1/1) contenant 10pmol de propranolol est sprayée à l'aide d'un système robotisé (SunCollect) sur les deux lots de dix lames ITO2 et ITO3 utilisées pour le calcul de la masse volumique.

Chaque coupe de tissu est ensuite analysée en imagerie MALDI à l'aide d'un Autoflex speed LRF (Bruker, Daltonics).

Les intensités du propranolol (m/z 260.2) de chaque lot de coupes sont moyennées sur la coupe de tissu et sur le support.

Le TEC peut alors être calculé en utilisant les valeurs de l'intensité du signal émis par le propranolol sur la lame et sur les tissus respectivement, selon la formule mathématique :

$$TEC = \frac{Int(lame)_X}{Int(tissu)_X}$$

Comme cela est repris dans le tableau 2, on constate que les valeurs moyennes de TEC obtenues pour le rein reconstitué et pour le rein intègre sont identiques.

Le rein reconstitué présente donc la même masse volumique et le même coefficient d'extinction tissulaire qu'un rein intègre.

Tableau 2 : Comparaison des masses volumiques et des TEC dans un rein reconstitué et un rein intègre.

|  | Coupe rein reconstitué | Coupe rein intègre |
|---|---|---|
| Pixels | 121637 | 229374.2 |
| Surface (mm$^2$) | 12.1637 | 22.93742 |
| Hauteur (mm) | 0.02 | 0.02 |
| Volume (mm$^3$) | 0.243274 | 0.4587484 |
| Poids (mg)/coupe | 0.17 | 0.305 |
| Masse volumique (mg/mm3) | 0.7 | 0.66 |
| TEC | **1.9361** | **1.922125** |
| Ecar type | 0.162580886 | 0.408923827 |
| % erreur TEC | 8.4 | 21.3 |

Le propranolol se comporte donc de manière identique dans un rein reconstitué et dans un rein intègre. L'utilisation d'un tissu reconstitué pour la préparation d'une gamme de dilution d'une molécule selon l'invention peut donc permettre par la suite de quantifier de manière fiable ladite molécule dans un tissu intègre correspondant.

EXEMPLE 2 : PREPARATION D'UNE GAMME DE DILUTION DE PROPRANOLOL POUR LE REIN

On dépose une coupe comportant la succession d'échantillons de broyat de rein, et donc présentant les 6 concentrations successives de propanolol (dans l'ordre présenté dans le tableau 1), sur un support (lame ITO1).

On spraye de la matrice MALDI DHB (40mg/mL méthanol/TFA 0.1% 1/1) sur la coupe de tissu à l'aide d'un système robotisé (SunCollect).

Chaque portion de la coupe représentant un échantillon de broyat de rein est ensuite analysée en imagerie par spectrométrie de masse à l'aide d'un Autoflex speed LRF (Bruker, Daltonics).

On délimite ensuite sur l'image obtenue par spectrométrie de masse pour chacun des échantillons de broyat de tissu plusieurs régions d'intérêt (ROI), d'égales dimensions.

On choisit d'utiliser comme signal de référence l'intensité des pics du spectre de masse. On récupère les intensités des pics du spectre de masse du propranolol pour chaque ROI, pour chacun des échantillons de broyat de tissu. Et pour chaque échantillon de broyat de tissu, on calcule une intensité moyenne (ROI moyen) à partir de l'intensité obtenue pour tous les ROIs correspondants.

Les intensités moyennes des pics du spectre de masse du propranolol pour chaque point de la gamme de dilution, c'est-à-dire pour chaque échantillon de broyat de tissu, sont répertoriées dans le tableau 3.

Tableau 3 : Intensités moyennes obtenues par imagerie MALDI pour chaque point de la gamme de dilution de propranolol (m/z 260.2) dans un rein reconstitué

| Numéro d'échantillon de broyat de rein | Concentration finale en propranolol dans le tissu reconstitué ($\mu$g/g) | Intensité |
|---|---|---|
| 1 | 2.8 | 9273.9 |
| 2 | 5.6 | 18223.0 |
| 3 | 11.2 | 37269.0 |
| 4 | 22.4 | 74191.0 |
| 5 | 44.8 | 145396.6 |
| 6 | 0.0 | 500.3 |

Une droite d'étalonnage peut alors être dessinée. Cette droite d'étalonnage peut être utilisée ultérieurement, lors d'un procédé de quantification du propranolol dans un rein. Il suffit de corréler l'intensité du pic du spectre de masse obtenue pour le propranolol dans le rein analysé à une concentration sur ladite droite de calibration.

Par exemple, on trace une droite de calibration représentant la concentration en propranolol dans le tissu ($\mu$g/g) en fonction de l'intensité du pic du spectre de masse (figure 2). L'équation de la courbe de tendance de l'ensemble des points de la gamme de dilution, ou nuage de points, est calculée sous le format y=ax+b à l'aide d'un logiciel de retraitement graphique, comme par exemple excel.

Dans l'exemple décrit, l'équation de la courbe de tendance est y = 3244,7x + 534,92. Cette équation permet par la suite la quantification de la molécule.

De même, on calcule le coefficient de détermination (ici $R^2$=0.9999) qui est un indicateur qui permet de juger la qualité d'une régression linéaire, simple ou multiple. Plus ce coefficient est proche de 1, plus la courbe de tendance est linéaire.

EXEMPLE 3 : QUANTIFICATION DU PROPRANOLOL DANS UN REIN DE SOURIS TRAITEE

Un rein de souris traitée à 7.5mg/kg de propranolol, et sacrifiée 20 minutes post-administration, est prélevé.

Trois coupes de tissu de 20$\mu$m d'épaisseur sont réalisées à partir du rein et sont déposées chacune sur un support (lame ITO).

De la matrice MALDI DHB (40mg/mL méthanol/TFA 0.1% 1/1) est sprayée à l'aide d'un système robotisé (SunCollect) sur les coupes.

Chaque coupe est analysée en imagerie par spectrométrie de masse, à l'aide d'un Autoflex speed LRF (Bruker, daltonics). L'intensité moyenne des pics du spectre de masse du propranolol (m/z 260.2) est calculée, en utilisant là encore plusieurs ROIs sur chaque coupe, tous de dimensions identiques.

La valeur de l'intensité moyenne obtenue est de 19633.3.

La concentration en propranolol dans le rein est quantifiée à l'aide de l'équation de la droite de calibration (figure 2), en reportant la valeur moyenne d'intensité obtenue sur ladite droite. Le propranolol est ici à une concentration de 5.9$\mu$g/g de tissu.

EXEMPLE 4 : EVALUATION DE LA LIMITE DE DETECTION DU PROPRANOLOL DANS LE REIN

Dans le cadre de cette utilisation, une gamme de dilution ou un point de dilution d'une ou plusieurs molécules d'intérêt dans des tissus reconstitués est utilisé(e) afin d'évaluer la détection de la ou des molécules à l'aide d'une méthode analytique choisie.

Dans cet exemple, on procède à la réalisation d'une gamme de dilution à partir de neuf sous-volumes précis différents de la solution de propranolol, qui sont chacun ajoutés à un échantillon de broyat de rein, de manière à obtenir une concentration dans les échantillons de 0, 0.3, 0.7, 1.4, 2.8, 5.6, 11.2, 22.4 et 44.8$\mu$g/g.

On dépose une coupe comportant la succession d'échantillons de broyat de rein, et donc présentant les 9 concentrations successives de propanolol (dans l'ordre présentée dans le tableau 4) sur un support (lame ITO).

Dans le cadre d'une analyse des coupes par imagerie MALDI-TOF, il est possible, grâce à l'invention, de mettre au point le protocole de préparation des tissus qui seront à analyser dans une phase préliminaire. En effet, il est possible d'utiliser la gamme préparée et d'effectuer plusieurs tests de préparation pour évaluer par exemple l'influence de l'épaisseur des coupes ou encore du choix de la matrice et/ou de son mode de dépôt sur le signal obtenu.

Dans cet exemple, l'épaisseur optimale est fixée à 20$\mu$m et la matrice MALDI la plus efficace est le DHB à 40mg/mL méthanol/TFA 0.1% 1/1.

Chaque portion de la coupe représentant un échantillon de broyat de rein est ensuite analysée en imagerie par spectrométrie de masse ou en analyse directe à l'aide d'un Autoflex speed LRF (Bruker, Daltonics).

Dans la cadre d'une acquisition en mode imagerie, on délimite sur l'image obtenue par spectrométrie de masse pour chacun des échantillons de broyat de tissu plusieurs régions d'intérêt (ROI), d'égales dimensions.

Dans le cadre d'une acquisition en mode analyse directe, on obtient un spectre moyen représentatif de chaque point de la gamme.

On choisit d'utiliser comme signal de référence l'intensité des pics du spectre de masse. On récupère les intensités des pics du spectre de masse du propranolol pour chacun des échantillons de broyat de tissu. Et pour chaque échantillon de broyat de tissu, on obtient donc une intensité moyenne.

Les intensités moyennes des pics du spectre de masse du propranolol pour chaque point de la gamme de dilution, c'est-à-dire pour chaque échantillon de broyat de tissu, sont répertoriées dans le tableau 4.

Tableau 4 : Intensités moyennes obtenues par imagerie MALDI pour chaque point de la gamme de dilution de propranolol (*m*/*z* 260.2) dans un rein reconstitué

| Numéro d'échantillon de broyat de rein | Concentration finale en propranolol dans le tissu reconstitué (μg/g) | Intensité |
|---|---|---|
| 1 | 0.0 | 500.3 |
| 2 | 0.3 | 520.6 |
| 3 | 0.7 | 2806.2 |
| 4 | 1.4 | 5077.5 |
| 5 | 2.8 | 9273.9 |
| 6 | 5.6 | 18223.0 |
| 7 | 11.2 | 37269.0 |
| 8 | 22.4 | 74191.0 |
| 9 | 44.8 | 145396.6 |

Dès lors, il est possible d'évaluer la limite de détection du composé dans le milieu choisi qui est ici de 0.7μg/g. Cette limite de détection est fixée dans cet exemple comme étant la concentration la plus basse pour laquelle le composé est détecté avec un ratio signal/bruit supérieur à 3. Notons qu'il est également possible de déterminer la limite de quantification.

Aussi si une quantification du composé dans un tissu traité devait être effectuée, on peut évaluer dans cet exemple que la méthode analytique est linéaire sur 3 log.

Plus généralement, l'invention permet d'évaluer plusieurs paramètres avant la préparation des systèmes d'essai in vivo, comme par exemple :

- L'épaisseur des coupes de tissu
- La matrice MALDI la plus adaptée pour la désorption et l'ionisation des composés
- Le mode de dépôt de la matrice
- La limite de détection et de quantification du ou des composés dans un milieu de référence
- La gamme de linéarité de la méthode analytique afin d'effectuer la quantification du ou des composés, etc.

EXEMPLE 5 : CONTROLE DE LA QUALITE ET DE LA REPRODUCTIBILITE D'ANALYSES EN IMAGERIE PAR SPECTROMETRIE DE MASSE MALDI

Dans cet exemple on souhaite évaluer la qualité et la reproductibilité d'une analyse en imagerie MALDI.

On dépose une coupe de broyat de rein comportant une concentration connue de propanolol (5.6μg/g) sur le même support (lame ITO) que l'échantillon d'intérêt. Notons que ce broyat de rein reconstitué est utilisé dans le cadre de toutes les analyses dont la reproductibilité est à évaluer.

Lors de chaque analyse des échantillons d'intérêt, pour lesquelles la même méthode de préparation et la même méthode analytique sont utilisées, une coupe du contrôle qualité est analysée en même temps en imagerie MALDI.

La valeur de l'intensité moyenne du propranolol est donc obtenue pour chaque analyse et il est ainsi possible de suivre la reproductibilité des analyses. Ainsi dans le cadre de 10 analyses de tissus d'intérêt, une coupe de rein reconstitué dopé avec du propranolol à 5.6μg/g a été ajouté. La figure 3 montre la variabilité obtenue dans le cadre de 10 analyses constitutives au sein d'une même étude.

Il est ainsi possible de suivre les variabilités expérimentales ou encore de normaliser les valeurs obtenues pour chaque analyse par rapport à ce contrôle qualité afin de faire de la quantification relative de(s) composé(s) d'intérêt entre différentes analyses.

Il est aussi possible de juger de la qualité de la préparation des tissus (plus particulièrement du dépôt de la matrice MALDI) et de la qualité des performances analytiques de l'instrument utilisé. Notons qu'une maintenance peut être déterminée en fonction des résultats de ces contrôles qualités. avec du propranolol à 5.6μg/g a été ajouté. La figure 3 montre la variabilité obtenue dans le cadre de 10 analyses constitutives au sein d'une même étude.

Il est ainsi possible de suivre les variabilités expérimentales ou encore de normaliser les valeurs obtenues pour chaque analyse par rapport à ce contrôle qualité afin de faire de la quantification relative de(s) composé(s) d'intérêt entre différentes analyses.

Il est aussi possible de juger de la qualité de la préparation des tissus (plus particulièrement du dépôt de la matrice MALDI) et de la qualité des performances analytiques de l'instrument utilisé. Notons qu'une maintenance peut être déterminée en fonction des résultats de ces contrôles qualités.

**Revendications**

1. Procédé de quantification d'au moins une molécule cible dans au moins un tissu biologique d'intérêt d'origine animale ou végétale comprenant les étapes suivantes :

   a) analyser une coupe d'un tissu d'intérêt, de manière à obtenir un signal représentatif de la quantité de molécule cible dans ladite coupe ;
   b) déterminer la quantité de molécule cible dans le tissu en utilisant une gamme de dilution d'une molécule étalon pour ledit tissu réalisée à partir de coupes d'un tissu reconstitué identique au tissu d'intérêt,

   dans lequel la gamme de dilution de la molécule étalon est obtenue selon les étapes consistant à :

   (i) broyer un tissu identique au tissu d'intérêt pour obtenir un broyat de tissu ;
   (ii) mélanger la molécule étalon à un premier échantillon du broyat de tissu de l'étape (i), ladite molécule étalon étant à une première concentration connue ;
   (iii) conditionner le premier échantillon de broyat de tissu issu de l'étape (ii) de manière à pouvoir couper ledit échantillon de broyat de tissu ;
   (iv) répéter les étapes (ii) et (iii) avec au moins un second échantillon du broyat de tissu de l'étape (i) et une seconde concentration connue de la molécule étalon, différente de la première concentration ;
   (v) analyser au moins une coupe de chacun des échantillons de broyat de tissu conditionné issu de l'étape (iii), de manière à obtenir un signal représentatif de la quantité de molécule étalon dans le tissu pour chacune des concentrations de ladite molécule étalon.

2. Procédé de quantification selon la revendication 1, dans lequel l'étape (iii) de conditionnement de l'échantillon de broyat de tissu consiste en une congélation et/ou en un enrobage dudit échantillon de broyat de tissu.

3. Procédé de quantification selon la revendication 1 ou 2, dans lequel l'étape (v) d'analyse consiste en une analyse par spectrométrie de masse, ou une analyse par fluorescence, ou une analyse par autoradiographie.

4. Procédé de quantification selon l'une des revendications 2 à 3, comprenant l'étape supplémentaire consistant à :
   (vi) homogénéiser l'échantillon de broyat de tissu et la molécule étalon de l'étape (ii) avant l'étape (iii) de conditionnement.

5. Procédé de quantification selon l'une des revendications 2 à 4, dans lequel l'échantillon de broyat conditionné ou reconstitué présente une masse volumique et/ou un coefficient d'extinction tissulaire identique à ceux du tissu correspondant intègre.

6. Procédé de quantification selon l'une des revendications 2 à 5, dans lequel on détecte et quantifie simultanément au moins deux molécules cibles différentes dans ledit tissu, la gamme étalon pour chacune des molécules cibles étant réalisée en utilisant pour l'étape (ii) de préparation de la gamme étalon au moins deux molécules étalons différentes qui sont ajoutées dans chacun des échantillons du broyat de tissu, l'analyse de l'étape (v) étant conduite pour chacune des molécules étalons de manière à obtenir le signal représentatif de la quantité de chacune des molécules étalons dans le tissu pour chacune des concentrations, et l'étape b) de détermination de la quantité de molécule cible étant conduite pour chacune des molécule cibles en utilisant la gamme de dilution correspondante et/ou dans lequel au moins deux tissus différents sont analysés, les étapes (i) à (v) de préparation d'une gamme étalon étant conduites pour chacun des tissus de manière à obtenir le signal représentatif de la molécule étalon pour chacun des tissus, l'étape b) de détermination de la quantité de molécule cible étant conduite pour chacun des tissus en utilisant la gamme de dilution correspondante.

7. Procédé de quantification selon l'une des revendications précédentes, dans lequel la molécule cible est une protéine, un peptide, un polypeptide, un acide aminé, un acide nucléique, un lipide, une drogue, une molécule active pharmaceutiquement ou biologiquement, ou un métabolite.

8. Procédé de quantification selon l'une des revendications précédentes, dans lequel l'étape a) consiste en une analyse par spectrométrie de masse, le signal associé au spectre de masse de la molécule cible correspondant à l'intensité du pic, l'aire du pic ou le ratio signal/bruit dudit spectre de masse

9. Procédé de quantification selon l'une des revendications précédentes, dans lequel la molécule étalon utilisée pour la préparation de la gamme de dilution correspond à la molécule cible.

10. Procédé de quantification selon l'une des revendications précédentes, dans lequel la molécule étalon utilisée pour la préparation de la gamme de dilution est la molécule cible marquée d'un isotope.

11. Procédé de quantification selon l'une des revendications précédentes, dans lequel on utilise l'imagerie par spectrométrie de masse, l'intensité du signal de la molécule cible permettant de visualiser simultanément la distribution et la concentration en la molécule cible dans la coupe dudit tissu et/ou **caractérisé en ce qu'**on détecte et quantifie la molécule cible directement sur une coupe d'un animal entier, de manière à comparer simultanément la distribution de ladite molécule cible dans différents tissus de l'animal, au moyen d'une gamme de dilution propre à chacun des tissus.

12. Procédé de quantification selon l'une des revendications précédentes, dans lequel l'étape b) de détermination de la quantité de molécule cible dans le tissu est exécutée par un système informatique, au moyen d'un support de données lisible par ordinateur comprenant des instructions exécutables par l'ordinateur adaptées pour permettre audit système informatique d'exécuter l'étape b).

13. Procédé de validation d'un procédé de détection et/ou de quantification d'au moins une molécule cible dans un tissu biologique d'intérêt d'origine animale ou végétale à partir d'une coupe dudit tissu, comprenant les étapes consistant à :

> (x) broyer un tissu identique au tissu auquel le procédé de détection et/ou de quantification doit être appliqué, pour obtenir un broyat de tissu ;
> (xi) mélanger une molécule étalon à au moins deux échantillons du broyat de tissu de l'étape (x), ladite molécule étalon étant à une même concentration connue pour les au moins deux échantillons de broyat de tissu ;
> (xii) conditionner les échantillons de broyat de tissu issus de l'étape (xi) de manière à pouvoir couper lesdits échantillons de broyat de tissu ;
> (xiii) analyser au moins une coupe de chacun des échantillons de broyat de tissu issu de l'étape (xii), de manière à obtenir un signal représentatif de la molécule étalon dans le tissu pour chacune des coupes ;
> (xiv) comparer les signaux obtenus pour chacune des coupes entre eux.

14. Procédé d'évaluation de la plus petite quantité d'une molécule cible détectable dans un tissu biologique d'intérêt d'origine animale ou végétale, selon lequel on identifie le plus petit signal détectable présentant une valeur signal/bruit supérieure ou égale à 3 à partir d'une gamme de dilution spécifique de la molécule cible et du tissu d'intérêt obtenue à partir de coupes d'un tissu reconstitué identique au tissu d'intérêt, selon lequel la gamme de dilution est obtenue selon les étapes consistant à :

> (i) broyer un tissu identique au tissu pour lequel on souhaite évaluer la plus petite quantité de la molécule cible détectable ou quantifiable, pour obtenir un broyat de tissu ;
> (ii) mélanger une molécule étalon à un premier échantillon du broyat de tissu de l'étape (i), ladite molécule étalon étant à une première concentration connue ;
> (iii) conditionner le premier échantillon de broyat de tissu issu de l'étape (ii) de manière à pouvoir couper ledit échantillon de broyat de tissu ;
> (iv) répéter les étapes (ii) et (iii) avec au moins un second échantillon du broyat de tissu de l'étape (i) et une seconde concentration connue de la molécule étalon, différente de la première concentration ;
> (v) analyser au moins une coupe de chacun des échantillons de broyat de tissu conditionné issu de l'étape (iii), de manière à obtenir un signal représentatif de la quantité de molécule étalon dans le tissu pour chacune des concentrations de ladite molécule étalon.

15. Procédé d'évaluation de la plus petite quantité d'une molécule cible quantifiable dans un tissu biologique d'intérêt d'origine animale ou végétale, selon lequel on identifie le plus petit signal présentant une valeur signal/bruit supérieure ou égale à 3 fois la valeur signal/bruit du plus petit signal détectable, à partir d'une gamme de dilution spécifique de la molécule cible et du tissu d'intérêt obtenue à partir de coupes d'un tissu reconstitué identique au tissu d'intérêt selon lequel la gamme de dilution est obtenue selon les étapes consistant à :

(i) broyer un tissu identique au tissu pour lequel on souhaite évaluer la plus petite quantité de la molécule cible détectable ou quantifiable, pour obtenir un broyat de tissu ;

(ii) mélanger une molécule étalon à un premier échantillon du broyat de tissu de l'étape (i), ladite molécule étalon étant à une première concentration connue ;

(iii) conditionner le premier échantillon de broyat de tissu issu de l'étape (ii) de manière à pouvoir couper ledit échantillon de broyat de tissu ;

(iv) répéter les étapes (ii) et (iii) avec au moins un second échantillon du broyat de tissu de l'étape (i) et une seconde concentration connue de la molécule étalon, différente de la première concentration ;

(v) analyser au moins une coupe de chacun des échantillons de broyat de tissu conditionné issu de l'étape (iii), de manière à obtenir un signal représentatif de la quantité de molécule étalon dans le tissu pour chacune des concentrations de ladite molécule étalon.

16. Procédé de contrôle de la qualité et/ou de la reproductibilité d'un procédé de détection et/ou de quantification d'au moins une molécule cible dans un tissu biologique d'intérêt d'origine animale ou végétale à partir d'une coupe dudit tissu, comprenant les étapes consistant à :

(xx) broyer un tissu identique au tissu d'intérêt, pour obtenir un broyat de tissu ;

(xxi) mélanger une concentration connue d'une molécule étalon à un échantillon du broyat de tissu de l'étape (xx) ;

(xxii) conditionner l'échantillon de broyat de tissu issu de l'étape (xxi) de manière à pouvoir couper ledit échantillon de broyat de tissu conditionné ;

(xxiii) pour chaque coupe du tissu d'intérêt à analyser, analyser simultanément une coupe de l'échantillon de broyat de tissu conditionné de l'étape (xxii), de manière à obtenir à chaque fois un signal représentatif de la molécule étalon pour la coupe de l'échantillon de broyat de tissu conditionné de l'étape (xxii) et un signal représentatif de la molécule cible pour le tissu d'intérêt ;

(xxiv) comparer entre eux les signaux obtenus pour chacune des coupes de l'échantillon de broyat de tissu conditionné de l'étape (xxii), ladite étape (xxiv) étant avantageusement exécutée par un système informatique, au moyen d'un support de données lisible par ordinateur comprenant des instructions exécutables par l'ordinateur adaptées pour permettre audit système informatique d'exécuter ladite étape (xxiv) et éventuellement l'étape de pondération utilisant les résultats selon l'étape (xxiv).

17. Procédé de quantification relative d'au moins une molécule cible dans un tissu biologique d'intérêt d'origine animale ou végétale, comprenant les étapes consistant à :

(xxx) broyer un tissu identique au tissu d'intérêt, pour obtenir un broyat de tissu ;

(xxxi) mélanger une concentration connue d'une molécule étalon à un échantillon du broyat de tissu de l'étape (xxx) ;

(xxxii) conditionner l'échantillon de broyat de tissu issu de l'étape (xxxi) de manière à pouvoir couper ledit échantillon de broyat de tissu conditionné ;

(xxxiii) pour chaque coupe du tissu d'intérêt à analyser, analyser simultanément une coupe de l'échantillon de broyat de tissu conditionné de l'étape (xxxii), de manière à obtenir à chaque fois un signal représentatif de la molécule étalon pour la coupe de l'échantillon de broyat de tissu conditionné de l'étape (xxxii) et un signal représentatif de la molécule cible pour le tissu d'intérêt ;

(xxxiv) calculer pour chaque coupe du tissu d'intérêt à analyser le ratio signal de la molécule cible dans le tissu d'intérêt sur signal de la molécule étalon dans le tissu reconstitué ;

(xxxv) comparer entre eux les ratios obtenus pour chaque coupe du tissu d'intérêt pour obtenir une quantification relative de la molécule cible,

ladite étape (xxxiv) et/ou ladite étape (xxxv) étant avantageusement exécutée par un système informatique, au moyen d'un support de données lisible par ordinateur comprenant des instructions exécutables par l'ordinateur adaptées pour permettre audit système informatique d'exécuter ladite étape (xxiv) et/ou ladite étape (xxxv).

18. Procédé de quantification selon la revendication 12, ou procédé de contrôle de la qualité et/ou de la reproductibilité selon la revendication 16, ou procédé de quantification relative selon la revendication 17, dans lequel le support de données lisible par ordinateur comporte une base de données de la gamme de dilution d'au moins une molécule étalon pour au moins deux tissus différents et/ou une base de données du signal de référence d'au moins une matrice utilisée en imagerie par spectrométrie de masse.

**Patentansprüche**

1. Quantifizierungsverfahren für mindestens ein Zielmolekül in mindestens einem interessierenden biologischen Gewebe tierischen oder pflanzlichen Ursprungs, umfassend die folgenden Schritte:

   a) Analysieren eines Schnitts eines interessierenden Gewebes, um ein Signal zu erhalten, das für die Menge des Zielmoleküls in diesem Schnitt repräsentativ ist;

   b) Bestimmen der Menge des Zielmoleküls im Gewebe unter Verwendung eines Verdünnungsbereichs eines Standardmoleküls für dieses Gewebe, das aus Schnitten eines rekonstituierten Gewebes hergestellt wurde, das mit dem interessierenden Gewebe identisch ist,

   wobei der Verdünnungsbereich des Standardmoleküls erhalten wird gemäß den Schritten umfassend:

   (i) Mahlen eines Gewebes, das mit dem interessierenden Gewebe identisch ist, um ein Gewebehomogenisat zu erhalten;

   (ii) Mischen des Standardmoleküls mit einer ersten Probe des Gewebehomogenisats aus Schritt (i), wobei dieses Standardmolekül eine erste bekannte Konzentration aufweist;

   (iii) Konditionieren der ersten Probe des Gewebehomogenisats, das aus Schritt (ii) resultiert, um in der Lage zu sein, diese Probe des Gewebehomogenisats schneiden zu können;

   (iv) Wiederholen der Schritte (ii) und (iii) mit mindestens einer zweiten Probe des Gewebehomogenats aus Schritt (i) und einer zweiten bekannten Konzentration des Standardmoleküls, die sich von der ersten Konzentration unterscheidet;

   (v) Analysieren mindestens eines Schnitts jeder der in Schritt (iii) konditionierten Proben des Gewebehomogenisats, um ein Signal zu erhalten, das für die Menge des Standardmoleküls im Gewebe für jede der Konzentrationen dieses Standardmoleküls repräsentativ ist.

2. Quantifizierungsverfahren nach Anspruch 1, wobei Schritt (iii) des Konditionierens der Probe des Gewebehomogenisats aus dem Einfrieren und/oder Beschichten dieser Probe des Gewebehomogenisats besteht.

3. Quantifizierungsverfahren nach Anspruch 1 oder 2, wobei Schritt (v) der Analyse aus einer Analyse durch Massenspektrometrie oder einer Analyse durch Fluoreszenz oder einer Analyse durch Autoradiographie besteht.

4. Quantifizierungsverfahren nach einem der Ansprüche 2 bis 3, umfassend den zusätzlichen Schritt:
   (vi) Homogenisieren der Probe des Gewebehomogenisats und des Standardmoleküls aus Schritt (ii) vor dem Konditionierungsschritt (iii).

5. Quantifizierungsverfahren nach einem der Ansprüche 2 bis 4, bei dem die Probe aus konditioniertem oder rekonstituiertem Homogenisat eine identische Dichte und/oder einen identischen Gewebeextinktionskoeffizienten wie das entsprechende intakte Gewebe aufweist.

6. Quantifizierungsverfahren nach einem der Ansprüche 2 bis 5, bei dem mindestens zwei verschiedene Zielmoleküle gleichzeitig in diesem Gewebe nachgewiesen und quantifiziert werden, wobei der Standardbereich für jedes der Zielmoleküle hergestellt wird, indem für Schritt (ii) zur Herstellung des Standardbereich mindestens zwei verschiedene Standardmoleküle verwendet werden, die jeder der Proben des Gewebehomogenisats zugesetzt werden, wobei die Analyse von Schritt (v) für jedes der Standardmoleküle durchgeführt wird, um das Signal zu erhalten, das für die Menge von jedem der Standardmoleküle im Gewebe für jede der Konzentrationen repräsentativ ist, und Schritt b) der Bestimmung der Menge des Zielmoleküls für jedes der Zielmoleküle mit dem entsprechenden Verdünnungsbereich durchgeführt wird und/oder bei dem mindestens zwei unterschiedliche Gewebe analysiert werden, wobei die Schritte (i) bis (v) des Vorbereitens eines Standardbereichs für jedes der Gewebe durchgeführt werden, um das Signal, das für das Standardmoleküls für jedes der Gewebe repräsentativ ist, zu erhalten, Schritt b) der Bestimmung der Menge des Zielmoleküls für jedes der Gewebe unter Verwendung des entsprechenden Verdünnungsbereichs durchgeführt wird.

7. Quantifizierungsverfahren nach einem der vorhergehenden Ansprüche, bei dem das Zielmolekül ein Protein, ein Peptid, ein Polypeptid, eine Aminosäure, eine Nukleinsäure, ein Lipid, ein Medikament, ein pharmazeutisch oder biologisch aktives Molekül oder ein Metabolit ist.

8. Quantifizierungsverfahren nach einem der vorhergehenden Ansprüche, bei dem Schritt a) aus einer Analyse durch Massenspektrometrie besteht, wobei das dem Massenspektrum des Zielmoleküls zugeordnete Signal der Intensität des Peaks, der Peakfläche oder dem Verhältnis Signal/Rauschen dieses Massenspektrums entspricht.

9. Quantifizierungsverfahren nach einem der vorhergehenden Ansprüche, bei dem das zur Erstellung des Verdünnungsbereichs verwendete Standardmolekül dem Zielmolekül entspricht

10. Quantifizierungsverfahren nach einem der vorhergehenden Ansprüche, bei dem das zur Erstellung des Verdünnungsbereichs verwendete Standardmolekül das mit einem Isotop markierte Zielmolekül ist.

11. Quantifizierungsverfahren nach einem der vorhergehenden Ansprüche, bei dem bildgebende Massenspektrometrie verwendet wird, wobei die Intensität des Signals des Zielmoleküls es ermöglicht, gleichzeitig die Verteilung und die Konzentration des Zielmoleküls im Schnitt dieses Gewebes sichtbar zu machen und/oder **dadurch gekennzeichnet, dass** das Zielmolekül direkt auf einem Schnitt eines ganzen Tieres nachgewiesen und quantifiziert wird, um so gleichzeitig die Verteilung des Zielmoleküls in verschiedenen Geweben des Tieres mittels eines für jedes Gewebe spezifischen Verdünnungsbereichs zu vergleichen .

12. Quantifizierungsverfahren nach einem der vorhergehenden Ansprüche, bei dem Schritt b) des Bestimmens der Menge des Zielmoleküls im Gewebe durch ein Computersystem mittels eines computerlesbaren Datenträgers durchgeführt wird, der angepasste computerausführbare Anweisungen umfasst, um dem Computersystem zu ermöglichen, Schritt b) auszuführen

13. Verfahren zur Validierung eines Verfahrens zum Nachweis und/oder zur Quantifizierung mindestens eines Zielmoleküls in einem interessierenden biologischen Gewebe tierischen oder pflanzlichen Ursprungs aus einem Schnitt des Gewebes, umfassend die Schritte bestehend aus:

(x) Mahlen eines Gewebes, das mit dem Gewebe identisch ist, auf das das Nachweis- und/oder Quantifizierungsverfahren angewendet werden soll, um ein Gewebehomogenisat zu erhalten;
(xi) Mischen eines Standardmoleküls mit mindestens zwei Proben des Gewebehomogenisats aus Schritt (x), wobei dieses Standardmolekül die gleiche bekannte Konzentration für die mindestens zwei Proben des Gewebehomogenisats aufweist;
(xii) Konditionieren der aus Schritt (xi) resultierenden Proben des Gewebehomogenisats, um in der Lage zu sein, diese Proben des Gewebehomogenisats schneiden zu können;
(xiii) Analysieren mindestens eines Schnitts jeder der Proben des Gewebehomogenisats aus Schritt (xii), um ein Signal zu erhalten, das für das Standardmolekül in dem Gewebe für jeden der Schnitte repräsentativ ist;
(xiv) Vergleichen der für jeden der Schnitte erhaltenen Signale untereinander.

14. Verfahren zur Bestimmung der kleinsten nachweisbaren Menge eines Zielmoleküls in einem interessierenden biologischen Gewebe tierischen oder pflanzlichen Ursprungs, bei dem das kleinste nachweisbare Signal mit einem Signal/Rausch-Wert größer oder gleich 3 unter Verwendung eines Verdünnungsbereichs spezifisch für das Zielmoleküls und das interessierenden Gewebes identifiziert wird, hergestellt aus Schnitten eines rekonstituierten Gewebes, das mit dem interessierenden Gewebe identisch ist, wobei der Verdünnungsbereich erhalten wird gemäß den Schritten umfassend:

(i) Mahlen eines Gewebes, das mit dem Gewebe identisch ist, für das die kleinste nachweisbare oder quantifizierbare Menge des Zielmoleküls bestimmt werden soll, um ein Gewebehomogenisat zu erhalten;
(ii) Mischen eines Standardmoleküls mit einer ersten Probe des Gewebehomogenisats aus Schritt (i), wobei dieses Standardmolekül eine erste bekannte Konzentration aufweist;
(iii) Konditionieren der ersten Probe des Gewebehomogenisats, das aus Schritt (ii) resultiert, um in der Lage zu sein, diese Probe des Gewebehomogenisats schneiden zu können;
(iv) Wiederholen der Schritte (ii) und (iii) mit mindestens einer zweiten Probe des Gewebehomogenats aus Schritt (i) und einer zweiten bekannten Konzentration des Standardmoleküls, die sich von der ersten Konzentration unterscheidet;
(v) Analysieren mindestens eines Schnitts jeder der in Schritt (iii) konditionierten Proben des Gewebehomogenisats, um ein Signal zu erhalten, das für die Menge des Standardmoleküls im Gewebe für jede der Konzentrationen dieses Standardmoleküls repräsentativ ist.

15. Verfahren zur Bestimmung der kleinsten quantifizierbaren Menge eines Zielmoleküls in einem interessierenden biologischen Gewebe tierischen oder pflanzlichen Ursprungs, bei dem das kleinste nachweisbare Signal mit einem Signal/Rausch-Wert größer oder gleich des 3-fachen des Signal/Rausch-Wert des kleinsten nachweisbaren Signals unter Verwendung eines Verdünnungsbereichs spezifisch für das Zielmoleküls und das interessierenden Gewebes identifiziert wird, hergestellt aus Schnitten eines rekonstituierten Gewebes, das mit dem interessierenden Gewebe identisch ist, wobei der Verdünnungsbereich erhalten wird gemäß den Schritten umfassend:

(i) Mahlen eines Gewebes, das mit dem Gewebe identisch ist, für das die kleinste Menge des nachweisbaren oder quantifizierbaren Zielmoleküls bestimmt werden soll, um ein Gewebehomogenisat zu erhalten;

(ii) Mischen eines Standardmoleküls mit einer ersten Probe des Gewebehomogenisats aus Schritt (i), wobei dieses Standardmolekül eine erste bekannte Konzentration aufweist;

(iii) Konditionieren der ersten Probe des Gewebehomogenisats, das aus Schritt (ii) resultiert, um in der Lage zu sein, diese Probe des Gewebehomogenisats schneiden zu können;

(iv) Wiederholen der Schritte (ii) und (iii) mit mindestens einer zweiten Probe des Gewebehomogenats aus Schritt (i) und einer zweiten bekannten Konzentration des Standardmoleküls, die sich von der ersten Konzentration unterscheidet;

(v) Analysieren mindestens eines Schnitts jeder der in Schritt (iii) konditionierten Proben des Gewebehomogenisats, um ein Signal zu erhalten, das für die Menge des Standardmoleküls im Gewebe für jede der Konzentrationen dieses Standardmoleküls repräsentativ ist.

16. Verfahren zur Kontrolle der Qualität und/oder der Reproduzierbarkeit eines Verfahrens zum Nachweis und/oder zur Quantifizierung mindestens eines Zielmoleküls in einem interessierenden biologischen Gewebe tierischen oder pflanzlichen Ursprungs aus einem Schnitt des Gewebes, umfassend die Schritte bestehend aus:

(xx) Mahlen eines Gewebes, das mit dem interessierenden Gewebe identisch ist, um ein Gewebehomogenisat zu erhalten;

(xxi) Mischen einer bekannten Konzentration eines Standardmoleküls mit einer Probe des Gewebehomogenats aus Schritt (xx);

(xxii) Konditionieren der Probe des Gewebehomogenats aus Schritt (xxi), um in der Lage zu sein, diese Probe des konditionierten Gewebehomogenisats schneiden zu können;

(xxiii) für jeden zu analysierenden Schnitt des interessierenden Gewebes, gleichzeitiges Analysieren eines Schnitts der in Schritt (xxii) konditionierten Probe des Gewebehomogenisats, um jedes Mal ein Signal zu erhalten, das für das Standardmolekül für den Schnitt des Probe des konditionierten Gewebehomogenisiats aus Schritt (xxii) repräsentativ ist, und ein Signal, das für das Zielmolekül für das interessierende Gewebe repräsentativ ist;

(xxiv) Vergleichen der Signale, die für jeden der Schnitte der Probe der konditionierten Gewebehomogenisate aus Schritt (xxii) erhaltenen werden, untereinander, wobei dieser Schritt (xxiv) vorteilhafterweise durch ein Computersystem mittels eines computerlesbaren Datenträgers durchgeführt wird, der angepasste computerausführbare Anweisungen umfasst, um dem Computersystem zu ermöglichen, Schritt (xxiv) auszuführen und optional den Schritt des Gewichtens unter Verwendung der Ergebnisse gemäß Schritt (xxiv) auszuführen.

17. Verfahren zur relativen Quantifizierung mindestens eines Zielmoleküls in einem interessierenden biologischen Gewebe tierischen oder pflanzlichen Ursprungs, umfassend die Schritte bestehend aus:

(xxx) Mahlen eines Gewebes, das mit dem interessierenden Gewebe identisch ist, um ein Gewebehomogenisat zu erhalten;

(xxxi) Mischen einer bekannten Konzentration eines Standardmoleküls mit einer Probe des Gewebehomogenats aus Schritt (xxx);

(xxxii) Konditionieren der Probe des Gewebehomogenats aus Schritt (xxxi), um in der Lage zu sein, diese Probe des konditionierten Gewebehomogenisats schneiden zu können;

(xxxiii) für jeden zu analysierenden Schnitt des interessierenden Gewebes, gleichzeitiges Analysieren eines Schnitts der in Schritt (xxxii) konditionierten Probe des Gewebehomogenisats, um jedes Mal ein Signal zu erhalten, das für das Standardmolekül für den Schnitt des Probe des konditionierten Gewebehomogenisiats aus Schritt (xxxii) repräsentativ ist, und ein Signal, das für das Zielmolekül für das interessierende Gewebe repräsentativ ist;

(xxxiv) Berechnen für jeden Schnitt des interessierenden Gewebes zum Analysieren des Signalverhältnisses des Zielmoleküls im interessierenden Gewebe zum Signal des Standardmoleküls im rekonstituierten Gewebe;

(xxxv) Vergleichen der Verhältnisse, die für jeden Schnitt des interessierenden Gewebes erhaltenen werden untereinander, um eine relative Quantifizierung des Zielmoleküls zu erhalten,

wobei dieser Schritt (xxxiv) und/oder dieser Schritt (xxxv) vorteilhafterweise durch ein Computersystem mittels eines computerlesbaren Datenträgers durchgeführt wird, der angepasste computerausführbare Anweisungen umfasst, um dem Computersystem zu ermöglichen, diesen Schritt (xxxiv) und/oder oder diesen Schritt (xxxv) auszuführen.

18. Quantifizierungsverfahren nach Anspruch 12, Verfahren zur Kontrolle der Qualität und/oder der Reproduzierbarkeit nach Anspruch 16 oder Verfahren zur relativen Quantifizierung nach Anspruch 17 bei dem der computerlesbare Datenträger eine Datenbank des Verdünnungsbereichs mindestens eines Standardmoleküls von mindestens zwei verschiedenen Geweben und/oder eine Datenbank des Referenzsignals mindestens einer Matrix, die bei der Bildgebung durch Massenspektrometrie verwendet wird.

**Claims**

1. Process for quantifying at least one target molecule in at least one biological tissue of interest of animal or plant origin comprising the following steps:

   a) analyzing a section of tissue of interest so as to obtain a signal representative of the quantity of target molecule in said section;
   b) determining the quantity of target molecule in the tissue by using a dilution range of a standard molecule for said tissue made from sections of a reconstituted tissue identical to the tissue of interest,

   wherein the dilution range of the standard molecule is obtained according to the steps consisting of:

   (i) grinding a tissue identical to the tissue of interest to obtain a ground tissue:
   (ii) mixing the standard molecule with a first sample of ground tissue of step (i), said standard molecule being at a first known concentration;
   (iii) conditioning the first ground tissue sample derived from step (ii) so as to be able to slice said ground tissue sample;
   (iv) repeating steps (ii) and (iii) with at least one second ground tissue sample of step (i) and a second known concentration of the standard molecule, different from the first concentration;
   (v) analyzing at least one section of each of the conditioned ground tissue samples derived from step (iii), so as to obtain a signal representative of the quantity of standard molecule in the tissue for each of the concentrations of said standard molecule.

2. Quantification process according to claim 1, wherein step (iii) of conditioning the ground tissue sample consists of freezing and/or coating said ground tissue sample.

3. Quantification process according to claim 1 or 2, wherein analysis step (v) consists of a mass spectrometry analysis or a fluorescence analysis or an autoradiography analysis

4. Quantification process according to one of claims 2 to 3, comprising the additional step consisting of:
   (vi) homogenizing the ground tissue sample and the standard molecule of step (ii) before conditioning step (iii).

5. Quantification process according to one of claims 2 to 4, wherein the conditioned or reconstituted ground tissue sample has a density and/or a tissue extinction coefficient identical to those of the corresponding intact tissue.

6. Quantification process according to one of claims 2 to 5, wherein at least two different target molecules are detected and quantified simultaneously in said tissue, the standard range for each of the target molecules being created by using, for step (ii) of preparing the standard range, at least two different standard molecules which are added to each of the ground tissue samples, analysis step (v) being conducted for each of the standard molecules so as to obtain the signal representative of the quantity of each of the standard molecules in the tissue for each concentration, and step b) of determining the quantity of target molecule conducted for each of the target molecules by using the corresponding dilution range and/or wherein at least two different tissues are analyzed, steps (i) to (v) of preparing a standard range being conducted for each of the tissues so as to obtain the signal representative of the standard molecule for each of the tissues, step b) of determining the quantity of target molecule being conducted for each of the tissues by using the corresponding dilution range.

7. Quantification process according to one of the preceding claims, wherein the target molecule is a protein, a peptide, a polypeptide, an amino acid, a nucleic acid, a lipid, a drug, a pharmaceutically- or biologically-active molecule or a metabolite.

8. Quantification process according to anyone of the preceding claims, wherein step a) consists of a mass spectrometry analysis, the signal associated with the mass spectrum of the target molecule corresponding to the peak intensity, the area of the peak or the signal/noise ratio of said mass spectrum.

9. Quantification process according to one of the preceding claims, wherein the standard molecule used for the preparation of the dilution range corresponds to the target molecule.

10. Quantification process according to one of the preceding claims, wherein the standard molecule used for the preparation of the dilution range is the target molecule labelled with an isotope.

11. Quantification process according to one of the preceding claims, wherein mass spectrometry imaging is used, the signal intensity of the target molecule making it possible to simultaneously visualize the distribution and concentration of the target molecule in the section of said tissue and/or **characterized in that** the target molecule is detected and quantified directly in a section of a whole animal, so as to simultaneously compare the distribution of said target molecule in different tissues of the animal, by means of a specific calibration range for each of the tissues.

12. Quantification process according to any one of the preceding claims, wherein step b) of determining the quantity of target molecule in the tissue is executed by a computer system, by means of a computer-readable data carrier comprising computer-executable instructions adapted to allow said computer system to execute step b).

13. Process for validating a process for detecting and/or quantifying at least one target molecule in a biological tissue of interest of plant or animal origin from a section of said tissue, comprising the steps consisting of:

(x) grinding a tissue identical to the tissue to which the detection and/or quantification process is to be applied, to obtain a ground tissue;
(xi) mixing a standard molecule with at least two samples of ground tissue from step (x), said standard molecule being at a same known concentration for the at least two ground tissue samples;
(xii) conditioning the ground tissue samples derived from step (xi) so as to be able to slice said ground tissue samples;
(xiii) analyzing at least one section of each of the ground tissue samples derived from step (xii), so as to obtain a signal representative of the quantity of standard molecule in the tissue for each of the of the sections;
(xiv) comparing the signals obtained for each of the sections to each other.

14. Process for evaluating the smallest quantity of a target molecule detectable in a biological tissue of interest of plant or animal origin, according to which the smallest detectable signal is identified having a signal/noise ratio greater than or equal to 3 from a specific dilution range for the target molecule and the tissue of interest obtained from reconstituted tissue sections identical to the tissue of interest, according to which the dilution range is obtained according to the steps consisting of:

(i) grinding a tissue identical to the tissue for which it is desired to evaluate the smallest quantity of the detectable or quantifiable target molecule, to obtain a ground tissue;
(ii) mixing a standard molecule with a first sample of ground tissue of step (i), said standard molecule being at a first known concentration;
(iii) conditioning the first ground tissue sample derived from step (ii) so as to be able to slice said ground tissue sample;
(iv) repeating steps (ii) and (iii) with at least one second ground tissue sample of step (i) and a second known concentration of the standard molecule, different from the first concentration;
(v) analyzing at least one section of each of the conditioned ground tissue samples derived from step (iii), so as to obtain a signal representative of the quantity of standard molecule in the tissue for each of the concentrations of said standard molecule.

15. Process for evaluating the smallest quantity of a target molecule quantifiable in a biological tissue of interest of plant or animal origin, according to which the smallest signal is identified having a signal/noise value greater than or equal to 3 times the signal/noise value of the smallest signal detectable, from a specific dilution range for the target molecule and the tissue of interest obtained from reconstituted tissue sections identical to the tissue of interest, according to which the dilution range is obtained according to the steps consisting of:

(i) grinding a tissue identical to the tissue for which it is desired to evaluate the smallest quantity of the detectable or quantifiable target molecule, to obtain a ground tissue;
(ii) mixing a standard molecule with a first sample of ground tissue of step (i), said standard molecule being at a first known concentration;
(iii) conditioning the first ground tissue sample derived from step (ii) so as to be able to slice said ground tissue sample;
(iv) repeating steps (ii) and (iii) with at least one second ground tissue sample of step (i) and a second known concentration of the standard molecule, different from the first concentration;
(v) analyzing at least one section of each of the conditioned ground tissue samples derived from step (iii), so as to obtain a signal representative of the quantity of standard molecule in the tissue for each of the concentrations of said standard molecule.

16. Process for control of the quality and/or reproducibility of a process for detecting and/or quantifying at least one target molecule in a biological tissue of interest of plant or animal origin from a section of said tissue, comprising the steps consisting of:

(xx) grinding a tissue identical to the tissue of interest to obtain a ground tissue;
(xxi) mixing a known concentration of a standard molecule with a ground tissue sample of step (xx);
(xxii) conditioning the ground tissue sample derived from step (xxi) so as to be able to slice said conditioned ground tissue sample;
(xxiii) for each of the tissues of interest to be analyzed, simultaneously analyzing a section of ground tissue conditioned in step (xxii), so as to obtain each time a signal representative of the standard molecule for the ground tissue sample section conditioned in step (xxii) and a signal representative of the target molecule for the tissue of interest;
(xxiv) comparing the signals obtained for each of the sections of the conditioned ground tissue sample from step (xxii), said step (xxiv) advantageously being carried out by means of a computer-readable data carrier comprising computer-executable instructions adapted to allow said computer system to execute step (xxiv) and optionally the step of weighting using the results according to step (xxiv).

17. Quantification process relative to at least one target molecule in a biological tissue of interest of plant or animal origin, comprising the steps consisting of:

(xxx) grinding a tissue identical to the tissue of interest to obtain a ground tissue;
(xxxi) mixing a known concentration of a standard molecule with a ground tissue sample of step (xxx);
(xxxii) conditioning the ground tissue sample derived from step (xxxi) so as to be able to slice said conditioned ground tissue sample;
(xxxiii) for each of the tissues of interest to be analyzed, simultaneously analyzing a section of ground tissue conditioned in step (xxxii), so as to obtain each time a signal representative of the standard molecule for the conditioned ground tissue sample section of step (xxxii) and a signal representative of the standard molecule for the tissue of interest;
(xxxiv) calculating for each section of the tissue of interest to be analyzed the signal ratio of the target molecule in the tissue of interest to the signal of the standard molecule in the reconstituted tissue;
(xxxv) comparing the ratios obtained for each of the tissues of interest to each other to obtain a relative quantification of the target molecule,
said step (xxxiv) and/or said step (xxxv) being advantageously executed by a computer system, by means of a computer-readable data carrier comprising computer-executable instructions adapted to allow said computer system to execute step (xxiv) and/or said step (xxxv).

18. Quantification process according to claim 12 or process for control of the quality and/or reproducibility according to claim 16, or relative quantification process according to claim 17, wherein the computer-readable data carrier comprises a database of the dilution range of at least one standard molecule for at least two different tissues and/or a database of the reference signal for at least one matrix used in mass spectrometry imaging.

Figure 1A

102

101

100

Figure 1B

101

201

200

FIGURE 1

$$y = 3244,7x + 534,92$$
$$R^2 = 0,9999$$

FIGURE 2

FIGURE 3

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **BECKER et al.** Elemental imaging mass spectrometry of thin sections of tissues and analysis of brain proteins in gels by laser ablation inductively coupled plasma mass spectrometry. *PHYSICA STATUS SOLIDI (C),* 2007, vol. 4 (6

- **HARE et al.** Quantitative elemental bio-imaging of Mn, Fe, Cu and Zn in 6-hydroxydopamine induced Parkinsonism mouse models. *METALLOMICS,* 2009, vol. 1 (1